# EUROPEAN PATENT APPLICATION

(11) **EP 1 878 803 A1**
(43) Date of publication of application: **16.01.2008**
(21) Application number: 06076383.6
(22) Date of filing: 10.07.2006
(51) Int. Cl.: C12Q 1/68

(54) **Methods of detecting root knot nematodes**

(71) Applicant: Wageningen Universiteit, 6701 BH Wageningen (NL); Bedrijfslaboratorium voor Grond- en Gewasonderzoek B.V., 6861 WN Oosterbeek (NL)
(72) Inventor: Helder, Johannes, 6707 AP Wageningen (NL); Karssen, Gerrit, 6721 JJ Bennekom (NL); van den Elsen, Sven Johannes Josephus, 5427 AW Boekel (NL); Holterman, Martijn Hermanus Maria, 6701 DH Wageningen (NL); Veenhuizen, Petrus Theodorus Maria, 6702 BS Wageningen (NL); Landeweert, Renske, 6862 VB Oosterbeek (NL); Hekman, Henri, 6862 VB Oosterbeek (NL); Bakker, Jaap, 6704 PD Wageningen (NL)
(74) Representative: van Loon, C.J.J.

(57) **Abstract**

The present invention relates to a method of testing a sample for the presence of meloidogyne nematodes, said method comprising analyzing the nucleic acid of said sample for the presence of a gene encoding a ribosomal RNA of a nematode or the transcription product thereof having a group-specific single nucleotide polymorphism (SNP) and/or a group-specific oligonucleotide polymorphism (OP), wherein said group is on a taxonomic level below that of the genus, but above that of the species, and optionally analyzing the nucleic acid of said sample for the presence of a gene encoding a ribosomal RNA of a nematode or the transcription product thereof having a species-specific single nucleotide polymorphism (SNP) and/or a species-specific oligonucleotide polymorphism (OP).

SEQUENCE LISTING

<110> Wageningen Universiteit
HELDER, Johannes
KARSSEN, Gerrit
VAN DEN ELSEN, Sven Johannes Josephus
HOLTERMAN, Martijn Herman Maria
VEENHUIZEN, Petrus Theodorus Maria

<120> Methods of detecting root knot nematodes

<130> P77469EP00

<140> EP 06076383.6
<141> 2006-07-10

<160> 49

<170> PatentIn version 3.3

<210> 1
<211> 74
<212> DNA
<213> Artificial

<220>
<223> SNP in SSU rDNA

<400> 1
aaaatctaga gctaatacat gcactaaagc tttgacctca cggaaaagcg catttattag 60

aacaaaacca cgcg 74


<210> 2
<211> 74
<212> DNA
<213> Artificial

<220>
<223> SNP in SSU rDNA

<400> 2
attagaacaa aaccacgcgg cttcggctgc tctttgttga ctcagaataa ctaagctgac 60

cgcatggcct tgyg 74


<210> 3
<211> 70
<212> DNA
<213> Artificial

<220>
<223> SNP in SSU rDNA

<400> 3
ttctggatgt tatcgattta tcgtaatgtt cagttttgag tccttarcag gattcttaac 60

aggcattgca 70


<210> 4
<211> 75
<212> DNA
<213> Artificial

<220>

<223>  SNP in SSU rDNA

<400>  4
tgagccattt cgagaaattt ggggactgtt gatttaactt ttttttaara agtttttttg      60

atggaaacca attta                                                      75


<210>  5
<211>  75
<212>  DNA
<213>  Artificial

<220>
<223>  SNP in SSU rDNA

<400>  5
attagaacaa aaccacgcgg cttcggctgc tttttgttga ctcagaataa ctaagctgac      60

cgcatggcct cwgtg                                                      75


<210>  6
<211>  71
<212>  DNA
<213>  Artificial

<220>
<223>  SNP in SSU rDNA

<400>  6
ttctggatgt tatcgatttt atcgtaatgt tcggttttga gtccttaaca ggattcttaa      60

caggcattgc a                                                          71


<210>  7
<211>  78
<212>  DNA
<213>  Artificial

<220>
<223>  SNP in SSU rDNA

<400>  7
actagcgatc cgccgatgga gattataatt gccttggtgg ggagcttccc ggaarcgaaa      60

gtcttccggt tccggggg                                                   78


<210>  8
<211>  76
<212>  DNA
<213>  Artificial

<220>
<223>  SNP in SSU rDNA

<400>  8
acgagcgaga ctctaaccta ctaaatagtt gatatttwtt tataaragwa tacaacttct      60

tagagggatt tgcggt                                                     76


<210>  9
<211>  70
<212>  DNA
<213>  Artificial

<220>
<223>  SNP in SSU rDNA

<400>  9
tgagccattt cgagaaattt ggggaccgtt rattaaacat ttatttgttt ttttgatgga      60

aaccaattta                                                              70


<210>  10
<211>  76
<212>  DNA
<213>  Artificial

<220>
<223>  SNP in SSU rDNA

<400>  10
acccactctc ggctcgagga ggtagtgacg agaaataacg agrtcgttct cwwatgaggc      60

cggtcatcgg aatggg                                                       76


<210>  11
<211>  75
<212>  DNA
<213>  Artificial

<220>
<223>  SNP in SSU rDNA

<400>  11
acgagcgaga ctctaaccta ctaaatagct gdtryataht hwahgtgtat wcrgcttctt      60

agagggattt gcggy                                                        75


<210>  12
<211>  75
<212>  DNA
<213>  Artificial

<220>
<223>  SNP in SSU rDNA

<400>  12
atacatagaa ttattgctgc ggttaraaag ctcrtagttg gattcgtatc grtaccytgg      60

aaccctycgg rgtgt                                                        75


<210>  13
<211>  77
<212>  DNA
<213>  Artificial

<220>
<223>  SNP in SSU rDNA

<400>  13
actagcgatc cgccgatgga aattatattg ccttggtggg gagcttcccg gaaacgaaag      60

tcttccggtt ccggggg                                                      77


<210>  14
<211>  75

<212>  DNA
<213>  Artificial

<220>
<223>  SNP in SSU rDNA

<400>  14
acgagcgaga ctctaaccta ctaaatagtt ggtrcatact cttwgtgtat acagcwtctt      60

agagggattt gcggc                                                        75


<210>  15
<211>  70
<212>  DNA
<213>  Artificial

<220>
<223>  SNP in SSU rDNA

<400>  15
tgagccattt cgagaaattt ggggaccgtt gatttaattt wtctaaatta ytttgatgga      60

arccaattta                                                              70


<210>  16
<211>  76
<212>  DNA
<213>  Meloidogyne chitwoodi

<400>  16
tagagtcggc gtatcttgca agtattcatt tttttttctg ttatattatt ttatcgctga      60

gctccagatt gggcag                                                       76


<210>  17
<211>  73
<212>  DNA
<213>  Meloidogyne chitwoodi

<400>  17
tgagacagat ttgtgaccac tattttgagg ccagctttgc tggtacccaa attgtgttag      60

catttttgt ttt                                                           73


<210>  18
<211>  77
<212>  DNA
<213>  Meloidogyne chitwoodi

<400>  18
atggcttacg ggcattattt ggtttgattt ggatgtaagt tacggtcgca tgcgrcacgt      60

gcttttcaat cagatcg                                                      77


<210>  19
<211>  76
<212>  DNA
<213>  Meloidogyne chitwoodi

<400>  19
gtcttgaaac acggaccaag gagtttatcg tgtgcgcaag ttattgggtg tttaaaacct      60

aaaagcgaaa tgaaag                                                       76

<210>  20
<211>  77
<212>  DNA
<213>  Meloidogyne fallax

<400>  20
tacatagaat atggcttacg ggcattattt ggtttgattt gggtgtaagt tacggtcgca     60

tgcgacacgt gcttttc                                                    77


<210>  21
<211>  69
<212>  DNA
<213>  Meloidogyne fallax

<400>  21
cttaagagtc tgatgtgcga tttctttttt ttaaaaggaa gcagcatggc cccattctaa     60

ttgtttaca                                                             69


<210>  22
<211>  76
<212>  DNA
<213>  Meloidogyne minor

<400>  22
agaaggtgca rgtcctgtac attgagatta ctggttaata tttgcgtgct tcagagtcgg     60

gttgcttggg atcgca                                                     76


<210>  23
<211>  74
<212>  DNA
<213>  Meloidogyne minor

<400>  23
agtattcatt tactttccgt tatattattt tattgctgag ctccagattg ggcagtgtaa     60

gattttatga ttga                                                       74


<210>  24
<211>  72
<212>  DNA
<213>  Meloidogyne minor

<400>  24
tgagacagat ttgtgacctc cattttgagg ccagcttgct ggtacccaag tggtgttagc     60

atttttgtt tt                                                          72


<210>  25
<211>  72
<212>  DNA
<213>  Meloidogyne minor

<400>  25
tgttagcatt ttttgttttg aatataaatt agagtatggc ttacgggcat taggtggttc     60

gatttgggtg ta                                                         72

<210>  26
<211>  78
<212>  DNA
<213>  Meloidogyne minor

<400>  26
agtaacggtc gcatgcgaca cgtgcttttc aactagatcg gtctatttaa tgctctcata      60

cttattcccc atgtaaaa                                                    78


<210>  27
<211>  77
<212>  DNA
<213>  Meloidogyne naasi

<400>  27
aacggcgagt gaactgggaa atgtccatcg ctaaatcatt ttgcctaagg cattatgagg      60

tgtagcgtat aagccgt                                                     77


<210>  28
<211>  77
<212>  DNA
<213>  Meloidogyne naasi

<400>  28
gatttttgat cagttttctc aaacaagtcc ccttgaacag gggttccaaa gaaggtgcaa      60

gtcctgtaca ttgagat                                                     77


<210>  29
<211>  79
<212>  DNA
<213>  Meloidogyne naasi

<400>  29
gcccaaagtc ggtggtaaac ttcatctaag actcaatatt gccacgartc cgatagcaaa      60

caagtactgt gaaggaaag                                                   79


<210>  30
<211>  76
<212>  DNA
<213>  Meloidogyne naasi

<400>  30
tggaaacgga tagagtcggc gtatcttgta agtattcatt tacattcagc tgtatttttt      60

agctttcgag ctccag                                                      76


<210>  31
<211>  75
<212>  DNA
<213>  Meloidogyne naasi

<400>  31
ttagctttcg agctccagat tgggacagag ttgaattata tgattgtttg tgatgcattt      60

acttgcttgg tgcgt                                                       75


<210>  32
<211>  73

<212>  DNA
<213>  Meloidogyne naasi

<400>  32
ttttgtttta gttgtaaaaa aagtatggct tttcgttcat tacgtggatc gttctgggtg      60

taagtaacgg tcg                                                          73


<210>  33
<211>  77
<212>  DNA
<213>  Meloidogyne hapla

<400>  33
atgtccatcg ctaaatcatt ttgcatcaaa catcatgagg tgtagcgtat aagccgtgat      60

ctttgatcag ttatctc                                                      77


<210>  34
<211>  76
<212>  DNA
<213>  Meloidogyne hapla

<400>  34
aaacaagtcc tcttgacaga ggttccaaag aaggtgcaag tcctgtacat tgggataact      60

gattgatatt tgcgta                                                       76


<210>  35
<211>  76
<212>  DNA
<213>  Meloidogyne hapla

<400>  35
aggacgtgaa accggtgagg aggaaacgga tagagtcggc gtatcttgca agtattcaat      60

tactttatta ttgtgt                                                       76


<210>  36
<211>  71
<212>  DNA
<213>  Meloidogyne hapla

<400>  36
atttacttgt ctggtgtgtg ggggtatcta agatggattt gcaaccaatg ttttgaggcc      60

agcttgctgg t                                                            71


<210>  37
<211>  68
<212>  DNA
<213>  Meloidogyne hapla

<400>  37
tgttaacatt ttttatcttg gatattcgag tacggcttac gtgcattttt tgtattgatc      60

taagtgca                                                                68


<210>  38
<211>  1753
<212>  DNA
<213>  Artificial

<220>
<223>  Consensus sequence of aligned SSU rDNA sequences (Sequence ID no:
       38)


<220>
<221>  VARIATION
<222>  (142)..(142)
<223>  t, c or g

<220>
<221>  VARIATION
<222>  (660)..(660)
<223>  t, c or g

<220>
<221>  VARIATION
<222>  (702)..(702)
<223>  t, c or g

<220>
<221>  VARIATION
<222>  (1047)..(1047)
<223>  t, c or g

<220>
<221>  VARIATION
<222>  (1351)..(1351)
<223>  t, c or g

<220>
<221>  VARIATION
<222>  (1357)..(1357)
<223>  t, c or g

<220>
<221>  VARIATION
<222>  (1691)..(1691)
<223>  t, c or g

<220>
<221>  VARIATION
<222>  (1703)..(1703)
<223>  t, c or g

<400>  38
atgtaymagt ttaatcghwt tgawdcgaga aaccgcgaac ggctcattac aatrgcyatd      60

atttacttga tcttgayywv tcctaahtgg ayaactgtgg aaaakctaga gctaatacat     120

gcachaaagc tyygwcccyt bavggggaaa ragcgcattt attdgacaca aaaccahgcr     180

gcttcggctg yyhhacgttt gttgactcag aataacthag ctgaccgcah ggcchhwghg     240

ccggcggcgt gtctttcaag ygtcydcttt atcatacttt cgayggkagy atmabygrct     300

mccrtggtkg tgacggataa cggagratma gggttcgact ccggagaagg ggcctgagaa     360

atggccacta cgtctaagga tggcagcagg cgcgcaaatt acccactctc ggcwytcaga     420

ggaggtagtg acgagaaata acragryhcg ttctcwwhwg aggccggtca tyggaatggg     480

tacaayttaa accctttaac gagtatcwab sagagggcaa gtctggtgac cagcagccgc     540

ggtaattcca gcttctsvda atrcatagra ttmttgctgc ggttaraaag ctcrtagttg     600

gactyhgwry ccgdtdcybt rgdccvccch ymggggtgtg ywctdvrtgt chgcaaybga     660

tttthybgya atgtwydgyy wdsvgbcctt aaacdggsad baydvrcagb crttrctgca 720

agtttacttt gaacaaatca gagtgcttca aacaggcgtw ytmgcttgaa tgwtcgtgca 780

tggaataata gaadabgatt tcggtcycdc trttttattg gttttayrga ctgagataat 840

ggttaahaga gacraaccgg gggcattygt atgghcmcgt gagaggtgaa attcttggac 900

cgtggccaga caractacag cgaaagcatt tgccaagary gtyttyatta atcaagaacg 960

aaagtcagag gttcgaaggm gatcagatac cgccctagtt ctgaccggta aacgatgcca 1020

actagcgatc cgcygatgga rtaythaaat tgcchtgggt ggggagcttc ccggaaacga 1080

aagtcttccg gttccggggg aagtatggtt gcaaagctga aacttaaagg aattgacgga 1140

agggcaccac caggagtgga gcctgcggct taatttgact caacacgggr aaactcaccc 1200

ggcccggaca cygtvaggat tgacagattg atagcttttt catgattcrg tggatggtgg 1260

tgcatggccg ttcttagttc gtggagtgat ttgtctggtt tattccgata acgagcgaga 1320

ctctvrccta ctaaaatagb bgdghrywtw atththagwr hgydydcvrc ttcttagagg 1380

gatttkcggy kttyagccgm amgaaattga gcaataacag gtctgtgatg cccttagatg 1440

tccggggctg cacgcgcgct acactggcaa aatcarcgtg cttgtccthc bcbgaaaggv 1500

ghkggctaaa ccmytgaaaa tttgccgtga ttgggatcgg aaattgcaat tattttccgt 1560

gaaccgagga attccaagta agtgcgagtc atcagctcgc gttgattacg tccctgccct 1620

ttgtacacac cgcccgtcgc tgcccgggac tgagccattt cgagaaaytt ggrgacygtt 1680

ratydksvry atgtttttha adaddtkhyt tttcgatgga arccaattta atcgcagyrg 1740

cttgaacccg ggc 1753

<210> 39
<211> 1069
<212> DNA
<213> Artificial

<220>
<223> Consensus sequence of aligned LSU rDNA sequences (Sequence ID no:
39)

<220>
<221> VARIATION
<222> (436)..(436)
<223> t, c or g

<220>
<221> VARIATION
<222> (640)..(641)
<223> t, c or g

<220>
<221> VARIATION
<222> (724)..(724)
<223> t, c or g

<400> 39
aagaaacgta aayakrgatt cgcyybrgat aacggcgagt gaamwgggaa agdgtccatc 60

gcywaatcrt tttgcmtmav rcrgtyrtga ggtgtagcgt rtaaghcgct rawytytgat 120
cdbwkdtcyc aaacaarttc cyyttgaaya grggttccah agaaggtgyr rgtcctgtac 180
vttgrgaygt wwytgrtyra taktygcgtd cttyargagt cgggttgyct tgggatcgca 240
gcccaaagtc ggtggtaaac ttcatctaag acybaatatt gccacgardc cgatagcawa 300
caaggtacyg tgarggaaag ttgcaaagca ctttgaagag agagttaaag aggacgtgaa 360
accggtragg wggaaackga tagagtcggc gtatcwtkya wktattccad ttdywtttyh 420
dytdtwkbdt bgtdraykyt cgagctccag attgggacag wgddrrdycw dyatrattdw 480
wttktrdtgc wtttaccttg hytggtgykt ggggdatdty tragryrgat ttgydhcydh 540
yrttttgagg ccagcctttc ggggtctggt acccrarydd brttarcawt ttttrctytt 600
rrdydtwctt tamwwhrart aayggcyhtw cgkdydtywa atrkwyhgwk ytrrdkggya 660
rgtwackgtc gcrtgcgaya mgtgcttttc rwyywkhtcr gtsyrkktwa tgyycycrta 720
ytyayccyyc ccatgtaama kccggtcatc tatyygaccc gtcttgaaac acggacccaa 780
ggagtttatc gtgtgcggcc aagttwttgg gtgttwaaaa ccytarargc gaaatgaaag 840
taaatgryyt cctthwggag tytgatgtgc rayhtyttyt ywwmkwhwdw awrawaagrw 900
hryakcatgg ccccattcta aytgtttaca rtagggtggm ggaagagcgt accgcggtga 960
gacccgaaag atggtgaact attcctgagc caggaygaag cccacgagga aactytggtg 1020
gaagtccgaa gcggytctga cgtgcaaatc gatcgtctga cttgggtat 1069

<210> 40
<211> 22
<212> DNA
<213> Artificial

<220>
<223> LSU rDNA-based primer to detect M. chitwoodi (forward)

<400> 40
gcattatttg gtttgatttg ga 22

<210> 41
<211> 22
<212> DNA
<213> Artificial

<220>
<223> LSU rDNA-based primer to detect M. fallax (forward)

<400> 41
gcattatttg gtttgatttg gg 22

<210> 42
<211> 25
<212> DNA
<213> Artificial

<220>
<223> LSU rDNA-based primer to detect M. minor (forward)

<400> 42

tcttgcaagt attcatttac tttcc                                          25

<210>  43
<211>  20
<212>  DNA
<213>  Artificial

<220>
<223>  LSU rDNA-based primer to detect M. naasi (forward)

<400>  43
ccagattggg acagagttga                                                20

<210>  44
<211>  21
<212>  DNA
<213>  Artificial

<220>
<223>  LSU rDNA-based primer to detect M. hapla (forward)

<400>  44
aagatggatt tgcaaccaat g                                              21

<210>  45
<211>  24
<212>  DNA
<213>  Artificial

<220>
<223>  LSU rDNA-based primer to detect M. chitwoodi (reverse)

<400>  45
gcttttaggt tttaaacacc caat                                           24

<210>  46
<211>  23
<212>  DNA
<213>  Artificial

<220>
<223>  LSU rDNA-based primer to detect M. fallax (reverse)

<400>  46
tggggccatg ctgcttcctt tta                                            23

<210>  47
<211>  24
<212>  DNA
<213>  Artificial

<220>
<223>  LSU rDNA-based primer to detect M. minor (reverse)

<400>  47
caaaacaaaa aatgctaaca ccac                                           24

<210>  48
<211>  20
<212>  DNA
<213>  Artificial

<220>
<223>  LSU rDNA-based primer to detect M. naasi (reverse)

<400>  48
acgatccacg taatgaacga                                                    20


<210>  49
<211>  20
<212>  DNA
<213>  Artificial

<220>
<223>  LSU rDNA-based primer to detect M. hapla (reverse)

<400>  49
caaaaaatgc acgtaagccg                                                    20

SEQUENCE LISTING

<110> Wageningen Universiteit
HELDER, Johannes
KARSSEN, Gerrit
VAN DEN ELSEN, Sven Johannes Josephus
HOLTERMAN, Martijn Herman Maria
VEENHUIZEN, Petrus Theodorus Maria

<120> Methods of detecting root knot nematodes

<130> P77469EP00

<140> EP 06076383.6
<141> 2006-07-10

<160> 49

<170> PatentIn version 3.3

<210> 1
<211> 74
<212> DNA
<213> Artificial

<220>
<223> Sequence ID no: 1

<400> 1
aaaatctaga gctaatacat gcactaaagc tttgacctca cggaaaagcg catttattag 60

aacaaaacca cgcg 74

<210> 2
<211> 74
<212> DNA
<213> Artificial

<220>
<223> Sequence ID no: 2

<400> 2
attagaacaa aaccacgcgg cttcggctgc tctttgttga ctcagaataa ctaagctgac 60

cgcatggcct tgyg 74

<210> 3
<211> 70
<212> DNA
<213> Artificial

<220>
<223> Sequence ID no: 3

<400> 3
ttctggatgt tatcgattta tcgtaatgtt cagttttgag tccttarcag gattcttaac 60

aggcattgca 70

<210> 4
<211> 75
<212> DNA
<213> Artificial

<220>
<223> Sequence ID no: 4

<400> 4
tgagccattt cgagaaattt ggggactgtt gatttaactt ttttttaara agtttttttg 60

atggaaacca attta 75


<210> 5
<211> 75
<212> DNA
<213> Artificial

<220>
<223> Sequence ID no: 5

<400> 5
attagaacaa aaccacgcgg cttcggctgc tttttgttga ctcagaataa ctaagctgac 60

cgcatggcct cwgtg 75


<210> 6
<211> 71
<212> DNA
<213> Artificial

<220>
<223> Sequence ID no: 6

<400> 6
ttctggatgt tatcgatttt atcgtaatgt tcggttttga gtccttaaca ggattcttaa 60

caggcattgc a 71


<210> 7
<211> 78
<212> DNA
<213> Artificial

<220>
<223> Sequence ID no: 7

<400> 7
actagcgatc cgccgatgga gattataatt gccttggtgg ggagcttccc ggaarcgaaa 60

gtcttccggt tccggggg 78


<210> 8
<211> 76
<212> DNA
<213> Artificial

<220>
<223>  Sequence ID no: 8

<400>  8
acgagcgaga ctctaaccta ctaaatagtt gatatttwtt tataaragwa tacaacttct      60

tagagggatt tgcggt                                                      76


<210>  9
<211>  70
<212>  DNA
<213>  Artificial

<220>
<223>  Sequence ID no: 9

<400>  9
tgagccattt cgagaaattt ggggaccgtt rattaaacat ttatttgttt ttttgatgga      60

aaccaattta                                                             70


<210>  10
<211>  76
<212>  DNA
<213>  Artificial

<220>
<223>  Sequence ID no: 10

<400>  10
acccactctc ggctcgagga ggtagtgacg agaaataacg agrtcgttct cwwatgaggc      60

cggtcatcgg aatggg                                                      76


<210>  11
<211>  75
<212>  DNA
<213>  Artificial

<220>
<223>  Sequence ID no: 11

<400>  11
acgagcgaga ctctaaccta ctaaatagct gdtryataht hwahgtgtat wcrgcttctt      60

agagggattt gcggy                                                       75


<210>  12
<211>  75
<212>  DNA
<213>  Artificial

<220>
<223>  Sequence ID no: 12

<400>  12

atacatagaa ttattgctgc ggttaraaag ctcrtagttg gattcgtatc grtaccytgg 60

aaccctycgg rgtgt 75

<210> 13
<211> 77
<212> DNA
<213> Artificial

<220>
<223> Sequence ID no: 13

<400> 13
actagcgatc cgccgatgga aattatattg ccttggtggg gagcttcccg gaaacgaaag 60

tcttccggtt ccggggg 77

<210> 14
<211> 75
<212> DNA
<213> Artificial

<220>
<223> Sequence ID no: 14

<400> 14
acgagcgaga ctctaaccta ctaaatagtt ggtrcatact cttwgtgtat acagcwtctt 60

agagggattt gcggc 75

<210> 15
<211> 70
<212> DNA
<213> Artificial

<220>
<223> Sequence ID no: 15

<400> 15
tgagccattt cgagaaattt ggggaccgtt gatttaattt wtctaaatta ytttgatgga 60

arccaattta 70

<210> 16
<211> 76
<212> DNA
<213> Artificial

<220>
<223> Sequence ID no: 16

<400> 16
tagagtcggc gtatcttgca agtattcatt tttttttctg ttatattatt ttatcgctga 60

gctccagatt gggcag 76

<210>  17
<211>  73
<212>  DNA
<213>  Artificial

<220>
<223>  Sequence ID no: 17

<400>  17
   tgagacagat ttgtgaccac tattttgagg ccagctttgc tggtacccaa attgtgttag      60
   cattttttgt ttt                                                          73


<210>  18
<211>  77
<212>  DNA
<213>  Artificial

<220>
<223>  Sequence ID no: 18

<400>  18
atggcttacg ggcattattt ggtttgattt ggatgtaagt tacggtcgca tgcgrcacgt      60

gcttttcaat cagatcg                                                     77


<210>  19
<211>  76
<212>  DNA
<213>  Artificial

<220>
<223>  Sequence ID no: 19

<400>  19
gtcttgaaac acggaccaag gagtttatcg tgtgcgcaag ttattgggtg tttaaaacct      60

aaaagcgaaa tgaaag                                                      76


<210>  20
<211>  77
<212>  DNA
<213>  Artificial

<220>
<223>  Sequence ID no: 20

<400>  20
tacatagaat atggcttacg ggcattattt ggtttgattt gggtgtaagt tacggtcgca      60

tgcgacacgt gcttttc                                                     77


<210>  21
<211>  69
<212>  DNA
<213>  Artificial

<220>
<223>  Sequence ID no: 21

<400>  21
cttaagagtc tgatgtgcga tttctttttt ttaaaaggaa gcagcatggc cccattctaa      60

ttgtttaca                                                              69


<210>  22
<211>  76
<212>  DNA
<213>  Artificial

<220>
<223>  Sequence ID no: 22

<400>  22
agaaggtgca rgtcctgtac attgagatta ctggttaata tttgcgtgct tcagagtcgg      60

gttgcttggg atcgca                                                      76


<210>  23
<211>  74
<212>  DNA
<213>  Artificial

<220>
<223>  Sequence ID no: 23

<400>  23
agtattcatt tactttccgt tatattattt tattgctgag ctccagattg ggcagtgtaa      60

gattttatga ttga                                                        74


<210>  24
<211>  72
<212>  DNA
<213>  Artificial

<220>
<223>  Sequence ID no: 24

<400>  24
   tgagacagat ttgtgacctc cattttgagg ccagcttgct ggtacccaag tggtgttagc      60
   attttttgtt tt                                                         72


<210>  25
<211>  72
<212>  DNA
<213>  Artificial

<220>
<223>  Sequence ID no: 25

<400>  25
tgttagcatt ttttgttttg aatataaatt agagtatggc ttacgggcat taggtggttc      60

gatttgggtg ta                                                          72


<210>  26
<211>  78
<212>  DNA
<213>  Artificial

<220>
<223>  Sequence ID no: 26

<400>  26
agtaacggtc gcatgcgaca cgtgcttttc aactagatcg gtctatttaa tgctctcata      60

cttattcccc atgtaaaa                                                    78


<210>  27
<211>  77
<212>  DNA
<213>  Artificial

<220>
<223>  Sequence ID no: 27

<400>  27
aacggcgagt gaactgggaa atgtccatcg ctaaatcatt ttgcctaagg cattatgagg      60

tgtagcgtat aagccgt                                                     77


<210>  28
<211>  77
<212>  DNA
<213>  Artificial

<220>
<223>  Sequence ID no: 28

<400>  28
gatttttgat cagttttctc aaacaagtcc ccttgaacag gggttccaaa gaaggtgcaa      60

gtcctgtaca ttgagat                                                     77


<210>  29
<211>  79
<212>  DNA
<213>  Artificial

<220>
<223>  Sequence ID no: 29

<400>  29
gcccaaagtc ggtggtaaac ttcatctaag actcaatatt gccacgartc cgatagcaaa      60

caagtactgt gaaggaaag                                                   79


<210>  30

<211>  76
<212>  DNA
<213>  Artificial

<220>
<223>  Sequence ID no: 30

<400>  30
tggaaacgga tagagtcggc gtatcttgta agtattcatt tacattcagc tgtatttttt      60

agctttcgag ctccag                                                      76


<210>  31
<211>  75
<212>  DNA
<213>  Artificial

<220>
<223>  Sequence ID no: 31

<400>  31
ttagctttcg agctccagat tgggacagag ttgaattata tgattgtttg tgatgcattt      60

acttgcttgg tgcgt                                                       75


<210>  32
<211>  73
<212>  DNA
<213>  Artificial

<220>
<223>  Sequence ID no: 32

<400>  32
ttttgtttta gttgtaaaaa aagtatggct tttcgttcat tacgtggatc gttctgggtg      60

taagtaacgg tcg                                                         73


<210>  33
<211>  77
<212>  DNA
<213>  Artificial

<220>
<223>  Sequence ID no: 33

<400>  33
atgtccatcg ctaaatcatt ttgcatcaaa catcatgagg tgtagcgtat aagccgtgat      60

ctttgatcag ttatctc                                                     77


<210>  34
<211>  76
<212>  DNA
<213>  Artificial

<220>

<223>  Sequence ID no: 34

<400>  34
aaacaagtcc tcttgacaga ggttccaaag aaggtgcaag tcctgtacat tgggataact      60

gattgatatt tgcgta                                                      76


<210>  35
<211>  76
<212>  DNA
<213>  Artificial

<220>
<223>  Sequence ID no: 35

<400>  35
aggacgtgaa accggtgagg aggaaacgga tagagtcggc gtatcttgca agtattcaat      60

tactttatta ttgtgt                                                      76


<210>  36
<211>  71
<212>  DNA
<213>  Artificial

<220>
<223>  Sequence ID no: 36

<400>  36
atttacttgt ctggtgtgtg ggggtatcta agatggattt gcaaccaatg ttttgaggcc      60

agcttgctgg t                                                           71


<210>  37
<211>  68
<212>  DNA
<213>  Artificial

<220>
<223>  Sequence ID no: 37

<400>  37
tgttaacatt ttttatcttg gatattcgag tacggcttac gtgcattttt tgtattgatc      60

taagtgca                                                               68


<210>  38
<211>  1753
<212>  DNA
<213>  Artificial

<220>
<223>  Consensus sequence of aligned SSU rDNA sequences (Sequence ID no:
       38)


<220>

<221>  misc_feature
<222>  (142)..(142)
<223>  n is a, c, g, or t

<220>
<221>  misc_feature
<222>  (660)..(660)
<223>  n is a, c, g, or t

<220>
<221>  misc_feature
<222>  (702)..(702)
<223>  n is a, c, g, or t

<220>
<221>  misc_feature
<222>  (1047)..(1047)
<223>  n is a, c, g, or t

<220>
<221>  misc_feature
<222>  (1351)..(1351)
<223>  n is a, c, g, or t

<220>
<221>  misc_feature
<222>  (1357)..(1357)
<223>  n is a, c, g, or t

<220>
<221>  misc_feature
<222>  (1691)..(1691)
<223>  n is a, c, g, or t

<220>
<221>  misc_feature
<222>  (1703)..(1703)
<223>  n is a, c, g, or t

<400>  38
atgtaymagt ttaatcghwt tgawdcgaga aaccgcgaac ggctcattac aatrgcyatd     60

atttacttga tcttgayywv tcctaahtgg ayaactgtgg aaaakctaga gctaatacat    120

gcachaaagc tyygwcccyt bnvggggaaa ragcgcattt attdgacaca aaaccahgcr    180

gcttcggctg yyhhacgttt gttgactcag aataacthag ctgaccgcah ggcchhwghg    240

ccggcggcgt gtctttcaag ygtcydcttt atcatacttt cgayggkagy atmabygrct    300

mccrtggtkg tgacggataa cggagratma gggttcgact ccggagaagg ggcctgagaa    360

atggccacta cgtctaagga tggcagcagg cgcgcaaatt acccactctc ggcwytcaga    420

ggaggtagtg acgagaaata acragryhcg ttctcwwhwg aggccggtca tyggaatggg    480

tacaayttaa accctttaac gagtatcwab sagagggcaa gtctggtgac cagcagccgc    540

ggtaattcca gcttctsvda atrcatagra ttmttgctgc ggttaraaag ctcrtagttg    600

gactyhgwry ccgdtdcybt rgdccvccch ymggggtgtg ywctdvrtgt chgcaaybgn    660

tttthybgya atgtwydgyy wdsvgbcctt aaacdggsad bnydvrcagb crttrctgca 720

agtttacttt gaacaaatca gagtgcttca aacaggcgtw ytmgcttgaa tgwtcgtgca 780

tggaataata gaadabgatt tcggtcycdc trttttattg gttttayrga ctgagataat 840

ggttaahaga gacraaccgg gggcattygt atgghcmcgt gagaggtgaa attcttggac 900

cgtggccaga caractacag cgaaagcatt tgccaagary gtyttyatta atcaagaacg 960

aaagtcagag gttcgaaggm gatcagatac cgccctagtt ctgaccggta aacgatgcca 1020

actagcgatc cgcygatgga rtaythnaat tgcchtgggt ggggagcttc ccggaaacga 1080

aagtcttccg gttccggggg aagtatggtt gcaaagctga aacttaaagg aattgacgga 1140

agggcaccac caggagtgga gcctgcggct taatttgact caacacgggr aaactcaccc 1200

ggcccggaca cygtvaggat tgacagattg atagcttttt catgattcrg tggatggtgg 1260

tgcatggccg ttcttagttc gtggagtgat ttgtctggtt tattccgata acgagcgaga 1320

ctctvrccta ctaaaatagb bgdghrywtw ntththngwr hgydydcvrc ttcttagagg 1380

gatttkcggy kttyagccgm amgaaattga gcaataacag gtctgtgatg cccttagatg 1440

tccggggctg cacgcgcgct acactggcaa aatcarcgtg cttgtccthc bcbgaaaggv 1500

ghkggctaaa ccmytgaaaa tttgccgtga ttgggatcgg aaattgcaat tattttccgt 1560

gaaccgagga attccaagta agtgcgagtc atcagctcgc gttgattacg tccctgccct 1620

ttgtacacac cgcccgtcgc tgcccgggac tgagccattt cgagaaaytt ggrgacygtt 1680

ratydksvry ntgtttttha adnddtkhyt tttcgatgga arccaattta atcgcagyrg 1740

cttgaacccg ggc 1753

<210> 39
<211> 1069
<212> DNA
<213> Artificial

<220>
<223> Consensus sequence of aligned LSU rDNA sequences (Sequence ID no:
39)

<220>
<221> misc_feature
<222> (436)..(436)
<223> n is a, c, g, or t

<220>
<221> misc_feature
<222> (640)..(641)
<223> n is a, c, g, or t

<220>

<221>  misc_feature
<222>  (724)..(724)
<223>  n is a, c, g, or t

<400>  39
aagaaacgta aayakrgatt cgcyybrgat aacggcgagt gaamwgggaa agdgtccatc      60

gcywaatcrt tttgcmtmav rcrgtyrtga ggtgtagcgt rtaaghcgct rawytytgat     120

cdbwkdtcyc aaacaarttc cyyttgaaya grggttccah agaaggtgyr rgtcctgtac     180

vttgrgaygt wwytgrtyra taktygcgtd cttyargagt cgggttgyct tgggatcgca     240

gcccaaagtc ggtggtaaac ttcatctaag acybaatatt gccacgardc cgatagcawa     300

caaggtacyg tgarggaaag ttgcaaagca ctttgaagag agagttaaag aggacgtgaa     360

accggtragg wggaaackga tagagtcggc gtatcwtkya wktattccad ttdywtttyh     420

dytdtwkbdt bgtdrnykyt cgagctccag attgggacag wgddrrdycw dyatrattdw     480

wttktrdtgc wtttaccttg hytggtgykt ggggdatdty tragryrgat ttgydhcydh     540

yrttttgagg ccagcctttc ggggtctggt acccrarydd brttarcawt ttttrctytt     600

rrdydtwctt tamwwhrart aayggcyhtw cgkdydtywn ntrkwyhgwk ytrrdkggya     660

rgtwackgtc gcrtgcgaya mgtgcttttc rwyywkhtcr gtsyrkktwa tgyycycrta     720

ytynyccyyc ccatgtaama kccggtcatc tatyygaccc gtcttgaaac acggacccaa     780

ggagtttatc gtgtgcggcc aagttwttgg gtgttwaaaa ccytarargc gaaatgaaag     840

taaatgryyt cctthwggag tytgatgtgc rayhtyttyt ywwmkwhwdw awrawaagrw     900

hryakcatgg ccccattcta aytgtttaca rtagggtggm ggaagagcgt accgcggtga     960

gacccgaaag atggtgaact attcctgagc caggaygaag cccacgagga aactytggtg    1020

gaagtccgaa gcggytctga cgtgcaaatc gatcgtctga cttgggtat               1069


<210>  40
<211>  22
<212>  DNA
<213>  Artificial

<220>
<223>  LSU rDNA-based primer to detect M. chitwoodi (forward)

<400>  40
gcattatttg gtttgatttg ga                                                22


<210>  41
<211>  22
<212>  DNA
<213>  Artificial

<220>
<223>  LSU rDNA-based primer to detect M. fallax (forward)

<400>  41
gcattatttg gtttgatttg gg                                                22

<210>  42
<211>  25
<212>  DNA
<213>  Artificial

<220>
<223>  LSU rDNA-based primer to detect M. minor (forward)

<400>  42
tcttgcaagt attcatttac tttcc                                             25

<210>  43
<211>  20
<212>  DNA
<213>  Artificial

<220>
<223>  LSU rDNA-based primer to detect M. naasi (forward)

<400>  43
ccagattggg acagagttga                                                   20

<210>  44
<211>  21
<212>  DNA
<213>  Artificial

<220>
<223>  LSU rDNA-based primer to detect M. hapla (forward)

<400>  44
aagatggatt tgcaaccaat g                                                 21

<210>  45
<211>  24
<212>  DNA
<213>  Artificial

<220>
<223>  LSU rDNA-based primer to detect M. chitwoodi (reverse)

<400>  45
gcttttaggt tttaaacacc caat                                              24

<210>  46
<211>  23
<212>  DNA
<213>  Artificial

<220>
<223>  LSU rDNA-based primer to detect M. fallax (reverse)

<400>  46
tggggccatg ctgcttcctt tta                                              23


<210>  47
<211>  24
<212>  DNA
<213>  Artificial

<220>
<223>  LSU rDNA-based primer to detect M. minor (reverse)

<400>  47
caaaacaaaa aatgctaaca ccac                                             24


<210>  48
<211>  20
<212>  DNA
<213>  Artificial

<220>
<223>  LSU rDNA-based primer to detect M. naasi (reverse)

<400>  48
acgatccacg taatgaacga                                                  20


<210>  49
<211>  20
<212>  DNA
<213>  Artificial

<220>
<223>  LSU rDNA-based primer to detect M. hapla (reverse)

<400>  49
caaaaaatgc acgtaagccg                                                  20

## Description

### FIELD OF THE INVENTION

The invention relates to methods for detecting root knot nematodes. In particular, the invention relates to methods for detecting nematode species of the genus *Meloidogyne,* more particularly those species that qualify as agricultural pests.

### BACKGROUND OF THE INVENTION

Root knot nematodes (RKN), or, in taxonomic terms, nematodes of the genus *Meloidogyne,* belong to the single most successful plant parasitic organisms of our planet. Their host range encompasses more than 1,000 plant species and their presence in crops affects both crop quality and crop production. This, in combination with their worldwide distribution, ranks them among the major plant pathogens.

The genus *Meloidogyne* comprises numerous species that are morphologically very alike. In geographic terms, the genus can be divided into species of tropical and of temperate climatic zones (hereinafter referred to as tropical and temperate species, respectively).

From an economic point of view the most relevant RKN species native to North-Western Europe are the temperate species *Meloidogyne fallax, M. chitwoodi, M. naasi, M. hapla* and *M. minor. M. fallax* and *M. chitwoodi* are classified as quarantine species by the European and Mediterranean Plant Protection Organization (EPPO), as they affect all major crops in North-Western Europe including potato, corn, sugar beet and tomato. A quarantine species is an official qualification for a plant parasitic species whose entry into an area is to be prevented and/or whose spread in an area is to be controlled due to its economic impact.

In principle, the climate of many important agricultural areas in the world, for instance that of North-Western European, is too cold for the 'tropical' root knot nematode species *M. incognita, M. javanica,* and *M. arenaria.* Nevertheless, populations of these species are known to establish themselves in greenhouses and the hydroponic cultivation systems based on closed circuit recirculation used therein allow infections with these RKN to spread rapidly. Serious economical damage can for instance be encountered in rose flower production. A sensitive detection system is therefore also needed for these species.

The possibilities for the monitoring and control of RKN are greatly affected by the organism's life cycle. The life cycle of RKN includes 4 juvenile stages (J-1 through J-4) and an adult life stage (♂ and ♀). RKN develop into J-2's within the egg. After hatching, the pre-parasitic J-2 is free living in soil in search of a suitable plant root. The J-2 penetrates the root, attaches to self-induced feeding cells, and reaches adulthood after three successive moults, either as male or female. The female remains immobilized and produces several hundreds of eggs, which - in case of sexual reproduction - are fertilized by free-living males. The embryos develop into J-1s, which subsequently moult into J-2s. Only two life stages can therefore be encountered outside the plant root: the J-2 and adult male.

In order to identify RKN that may be present on a commodity, it is necessary to extract specimens from the roots, tubers or from the surrounding soil (growing medium or substratum in greenhouses). In practice this means that a sample must be searched under the microscope for the presence of the species of interest. A typical procedure starts with the isolation of J2's or males from soils, or females from plant tissues by means of a dissecting microscope. For microscopic identification, specimens are examined mounted in fixative. Identification to species level is based on morphological characteristics and is tentative at best. The number of informative morphological characteristics of juveniles is very limited when compared to adults, which hampers the microscopic identification of the RKN, especially in soils. Microscopic identification of RKN is therefore laborious, time consuming and can be performed only by highly trained experts since it is based on a few subtle morphological characteristics of nematode worms that form a rare occurrence within a huge background of non-target nematodes.

Moreover, in official programs, confirmation of a positive identification for the presence of *M. chitwoodi* or *M. fallax* is required by using a dedicated isozyme test. The isozyme test for confirmation of RKN identification is based on the relative movements (RM%) of the individual isozymes malate dehydrogenase (Mdh, EC 1.1.1.37) and esterase (Est, EC 3.1.1.1) of young egg-laying *Meloidogyne* females in an electrophoresis gel.

If young egg-laying females cannot be detected and isolated, one has to try and detect second-stage juveniles, males or even eggs in the soil sample. As indicated, above, that is no easy task, and may result in false negative test results.

One way of circumventing the problem of morphological identification is to use PCR methods. The presently available PCR methods are based on detecting *M. chitwoodi-* or *M.* fallax-specific sequence differences in either the internal transcribed spacers (ITS) or the intergenic spacer (IGS), located, respectively, between and at the end of the repeating array of genes in the ribosomal DNA gene locus. Since these regions do not encode any product, they can evolve at a faster rate than the ribosomal coding regions, and the increased level of variation in these regions, compared to the rRNA genes themselves, makes them in principle suitable for detecting genetic variation within a genus, and to distinguish between species. However, the available tests are restricted to *M. chitwoodi* and *M. fallax* and they are virtually useless when performed in soil that contains a myriad of different nematodes with unknown responses to these tests.

Thus, in summary there are a number of problems associated with the prior art methods for detecting and identifying RKN in sample material:
- The detection of the RKN and thus of its associated disease in plant material by microscopic detection methods is hampered by the scarcity of informative morphological characteristics between the species in general and between the juvenile stages in particular. The morphological differences with other RKN are so subtle, that only expert eyes can spot the odd *M. chitwoodi* or M. *fallax* in a soil sample of can distinguish *M. chitwoodi* from M. *fallax.*
- The detection of the RKN and thus of its associated disease in soil material is hampered by the scarcity of the causative agents in the soil. RKN spent only a very small part of their life cycle in soil and natural soil nematode populations are dominated by bacterivorous and fungivorous nematodes. As a result, the sporadic RKN target must be detected among thousands of non-target organisms.
- If microscopic methods indicate the possible presence of *M. chitwoodi* or *M. fallax* in plant or soil material, isozyme test are required for confirmation. This lengthens an already lengthy analysis.
- The presently available PCR methods are restricted to the detection of either *M. chitwoodi* or *M. fallax,* and are useless in crude soil samples.

It is an aim of the present invention to provide a detection system for RKN, and more in particular a system for the detection of specific species of RKN, as such resolution is required for compliance with government regulations aiming to control transport of material which may pose a threat to agriculture. Therefore, it is an aim of the present invention to provide a test for all economically important species of RKN, not limited to detection of *M. chitwoodi* or *M. fallax.*

It is another aim of the present invention to provide a detection system that can be applied to any sample, including nucleic acid samples from unidentified nematodes, root or tubers of crops, and even soil.

It is still another object of the present invention to provide a reliable and reproducible detection method that can be performed fully independent from sample screenings based on morphological features of nematodes, and which can be performed by persons of average skill.

It is yet another aim of the present invention to provide a test system that is fast, and provides sufficient sensitivity to detect very low numbers of target RKN in a sample, thus preventing false-negative test results.

It is still a further aim to provide a test system that is specific and does not suffer from cross-reactivity with non-target materials, for instance with other free-living soil nematodes present in a sample, thus preventing false-positive test results.

### SUMMARY OF THE INVENTION

While the prior art methods test a sample for the presence of species-specific ITS or IGS sequences or for the presence of a species-specific isoenzyme profiles, the present invention now solves the above problems by providing a method of testing a sample wherein the presence of groups of meloidogyne nematodes is tested based on sequence similarities in their ribosomal RNA genes. This diverging approach, however, has as an effect that species specific identification possibilities unexpectedly emerge due to the possibility to compare the test result of a subsequently performed species-specific detection, now based on sequence differences in those genes, with the results of the group-specific test.

Thus, the present inventors have now discovered that, contrary to expectation, ribosomal RNA gene sequence data provide a very powerful basis for the development of a method of testing a sample for the presence of the economically most important species of meloidogyne nematodes, i.e. those species that cause the greatest harm to agriculture. It has now been found that a very advantageous testing system can be provided wherein the test, instead of detecting a single species, is based on detection of a group of nematodes consisting of a group of target species. Thus, based on comparative sequence analysis between known RKN pests and other nematodes, a very reliable detection method for certain groups of root knot nematodes could be developed in which cross-reactivity with non-target (background) populations could essentially be prevented, thus providing a test system of high specificity.

This finding was unexpected since many RKN species reproduce asexually or clonally (females produce daughters from unfertilized eggs), which results in a very high level of intra-species differentiation, wherein large differences can exist between separated populations of one and the same species. For instance, the genome of the tropical RKN species *Meloidogyne incognita* can be diploid (2n), triploid (3n) or tetraploid (4n), and her chromosome numbers can range between 32 and 46. With such high inter-species variation, the level of inter-species variation can be expected to be even higher. Therefore, it cannot be predicted whether molecular features can be found that are either unique to the species, or shared between species but unique to a group of species, while they are completely absent in other nematodes.

As stated above, while the present method is essentially based on the detection of groups of species, instead of individual species, this does in no way limit the application of the method for the detection of individual species of RKN. On the contrary, by combining the test method with an additional test having a resolution at the species level, it may be determined which species was initially detected by using the group-detection approach. An important additional advantage of a detection system with such a step-wise increase in resolution is that the second test may be used for confirmation purpose in case the first is positive. The details of this combination of tests will be described in more detail below.

In a first aspect, the present invention now provides a method of testing a sample for the presence of a meloidogyne nematode, said method comprising the step of analyzing the nucleic acid of a sample for the presence of a gene encoding a ribosomal RNA of a meloidogyne nematode or the transcription product thereof having a group-specific single nucleotide polymorphism (SNP) and/or a group-specific oligonucleotide polymorphism (OP), wherein said group comprises at least one species of a meloidogyne nematode of interest, and optionally analyzing the nucleic acid of said sample for the presence of a gene encoding a ribosomal RNA of a meloidogyne nematode or the transcription product thereof having a species-specific single nucleotide polymorphism (SNP) and/or a species-specific oligonucleotide polymorphism (OP), wherein said species is a meloidogyne nematode species of interest.

The suitability of a polymorphism in an rRNA gene sequence for the present purpose is primarily determined by its ability to reliably distinguish an arbitrary group of RKN species comprises at least one species of interest, preferably selected from the group consisting of *M. arenaria, M. chitwoodi, M. fallax, M. hapla, M incognita, M. javanica, M minor* and *M. naasi,* from other free-living nematodes. It was found that polymorphic nucleotide positions in the rRNA gene can be selected that separate the economically most important RKN species from the majority of free-living nematodes. Such an approach would not have been feasible based on ITS or IGS sequence data.

Since phylogenetic analyses based on DNA sequence data utilize a similar principle for clustering of genes according to similarity, the groups may suitably be selected according to phylogenetic lineage. However, this is not essential. For instance, certain polymorphism may be common among the meloidogyne nematodes and thus at first site appear of little value. Yet, they may provide discriminatory power towards other free-living nematodes. A more detailed explanation of the selection of suitable polymorphisms is provided below.

It was found that a very suitable first group comprises the species *M. chitwoodi, M. fallax* and *M. minor.* Thus, in a preferred embodiment the method is based on the detection of group-specific single nucleotide polymorphisms (SNPs) and/or group-specific oligonucleotide polymorphisms (OPs), i.e. polymorphisms that *M. chitwoodi, M. fallax* and *M*. *minor* have in common.

A surprising effect of the present invention is now that while this method does not attempt to detect individual species (e.g. *M. chitwoodi* or *M. fallax*), it is by virtue of the partitioning polymorphisms among a number of species, which polymorphisms set these species apart from free-living nematodes, that such species can be reliably detected with high specificity and sensitivity among the background of free-living nematodes in a soil sample. The groups need not necessarily comprise only RKN, nor do they necessarily need to contain only economically important RKN for the test to be of value. Rather, it is essential to provide a reliable indication of the presence or absence of a relatively small group of organisms which includes one or more RKN species of interest, to the exclusion of others. Subsequently, if necessary, a more detailed test may follow, in order to identify which species of the group is responsible for the positive test result.

While a first group may for instance comprises the species *M*. *chitwoodi, M. fallax* and *M. minor,* a second group may suitably comprise the species *M. naasi, M. oryzae* and *M. graminicola,* A third group may suitably comprise the species *M. hapla, M. microtyla, M. ardenensis, M. maritime* and *M. duytsi,* while a fourth group may suitably comprise the species *M. incognita, M. arenaria* and *M. jauarcica.* In this way a system can be developed with which the economically most important RKN can be detected by testing for only 4 different groups of RKN species, optionally followed by one or more species-specific test.

In a preferred embodiment, the group-specific single nucleotide polymorphism (SNP) and/or group-specific oligonucleotide polymorphism (OP) is a polymorphisms in the sequence of the small subunit (SSU or 18S) ribosomal RNA gene. Thus, the group-level resolution test is preferably based on SSU rRNA gene sequence data.

In another preferred embodiment, the species-specific single nucleotide polymorphism (SNP) and/or species-specific oligonucleotide polymorphism (OP) is a polymorphisms in the sequence of the large subunit (LSU; 28S and/or 5.8S) ribosomal RNA gene sequence. Thus, the species-level resolution test is preferably based on LSU rRNA gene sequence data. The one of average skill in the art will appreciate that the group-detection may also be based on LSU rRNA gene sequence data, while the species detection test may for some applications be based on SSU rRNA gene sequence data. Alternatively, both tests may be based on gene sequence data of the same ribosomal subunit.

In a particularly preferred embodiment of a method of the present invention, the method comprises the step of analyzing the nucleic acid of said sample for the presence of a gene encoding the SSU rRNA of a meloidogyne nematode or its transcription product having at least one single nucleotide polymorphism (SNP) and/or at least one oligonucleotide polymorphism (OP) specific for a group of meloidogyne nematodes comprising at least one species selected from the group consisting of *M. arenaria, M. chitwoodi, M. fallax, M. hapla, M. incognita, M. javanica, M. minor* and *M. naasi,.*

In a highly preferred embodiment, said polymorphism is indicative of:
(a) the group comprising the species *M. chitwoodi, M. fallax* and *M. minor,* wherein said polymorphism is selected from the group of polymorphisms consisting of:
   - OP: 141C, 142A, 143C, 151A, 152A, 153G of SEQ ID NO: 1;
   - OP : 192T, 193C, 194T, 199T, 200T, 202T of SEQ ID NO: 2;
   - SNP: 677A of SEQ ID NO: 3; and
   - OP: 1673G, 1677T of SEQ ID NO: 4;
   and/or
(b) the group comprising the species *M. naasi, M. oryzae* and *M. graminicola,* wherein said polymorphism is selected from the group of polymorphisms consisting of:
   - OP: 192T, 193T, 194T of SEQ ID NO: 5;
   - SNP: 664T of SEQ ID NO: 6;
   - OP: 1043A, 1045T, 1046A, 1049A of SEQ ID NO: 7;
   - OP: 1353T, 1354A, 1355T, 1356A, 1357A of SEQ ID NO: 8;
   - SNP: 1360G of SEQ ID NO: 8; and
   - OP: 1702T, 1703T, 1704T of SEQ ID NO: 9;
   and/or
(c) the group comprising the species *M. hapla, M. microtyla, M. ardenensis, M. maritime* and *M. duytsi,* wherein said polymorphism is selected from the group of polymorphisms consisting of:
   - SNP: 458A of SEQ ID NO: 10; and
   - OP: 1331C, 1340C, 1341T, 1342G of SEQ ID NO: 11;
   and/or
(d) the group comprising the species *M*. *incognita, M. arenaria* and *M*. *javanica,* wherein said polymorphism is selected from the group of polymorphisms consisting of:
   - OP: 617C, 620T, 623A, 626A of SEQ ID NO: 12;
   - SNP: 1041A of SEQ ID NO: 13;
   - OP: 1348A, 1349T, 1350A, 1351C, 1354C, 1355T, 1356T of SEQ ID NO: 14; and
   - OP: 1677C, 1679T, 1682A, 1683T, 1684T, 1685T of SEQ ID NO: 15; and wherein the presence in said gene or said transcription product of said polymorphism is indicative of the presence in said sample of at least one member of said group of the meloidogyne nematode.

In a preferred embodiment of the above-described method, said testing comprises the steps of (a) hybridizing an oligonucleotide comprising a nucleotide sequence complementary to at least a part of the SSU rRNA gene of a meloidogyne nematode, a transcription product thereof, or a complementary nucleic acid thereof under stringent conditions to said nucleic acid in said sample, wherein said oligonucleotide is complementary in the position of at least one group-specific polymorphism as defined herein, and (b) detecting whether hybridization has occurred, for instance by detecting the formation of stable hybrids between said oligonucleotide and said nucleic acids in said sample, wherein hybridization of said oligonucleotide is indicative for the presence of at least one member of said group of the meloidogyne nematode.

In a preferred embodiment of such a method, the oligonucleotide is detectably labelled. More preferably, the oligonucleotide is labelled with a reporter dye and a quenching dye.

In another preferred embodiment of a method of the invention, said testing comprises determining the nucleic acid sequence of at least a portion of said a gene encoding the SSU rRNA of a nematode or its transcription product that comprises the group-specific polymorphism. Preferably, such determination of the nucleic acid sequence is effected by solid-phase minisequencing.

In another preferred embodiment of a method of the invention, said method further comprises the amplification of at least a portion of said nucleic acid, preferably prior to analysing the nucleic acid.

The oligonucleotide comprising the polymorphic position as used in a method of the invention may be a probe, but is preferably an amplification primer. In such instances, the detection of hybridization in step (b) as described above comprises performing a nucleic acid amplification reaction with said amplification primer.

Preferred nucleic acid amplification reactions used in methods of the present invention encompass quantitative PCR, more preferably "real time" quantitative PCR.

In another embodiment of a method of the invention prior to or instead of analyzing the nucleic acid, at least a portion of said nucleic acid is amplified by a nucleic acid amplification reaction, the reaction comprising cycles of:
(a) stringent hybridization with at least two primers sufficiently complementary to bind to said nucleic acid under stringent conditions of hybridization, wherein at least one primer is complementary to the SSU rRNA gene of a group of meloidogyne nematodes comprising at least one species of interest or of its corresponding transcription product or a complement thereof in the position of at least one polymorphism that is specific for said group, and
(b) strand elongation and denaturing,
wherein failure to generate an amplification product is indicative for the absence of a member of said group of the meloidogyne nematode comprising the polymorphism, and the generation of an amplification product is indicative for the presence of a member of said group of the meloidogyne nematode comprising the polymorphism.

In one highly preferred embodiment the sample used in a method of the present invention is a soil sample.

In another highly preferred embodiment, the sample used in a method of the present invention is a root or tuber sample.

In yet another highly preferred embodiment, the sample used in a method of the present invention is a nucleic acid sample isolated from a nematode. In such an embodiment, the method relates to identification.

The method of the present invention may be extended by performing more detailed analysis of the sample. For instance upon positive detection of a member of said group of the meloidogyne nematode in said sample said method further comprises the step of testing said sample for the presence of at least one species of meloidogyne nematode selected from the species *M*. *chitwoodi, M. fallax, M. minor, M. naasi* and *M. hapla.*

Said method may for instance comprise:
- additionally analyzing the nucleic acid of said sample for the presence of a gene encoding the LSU rRNA of a nematode or its transcription product having a species-specific single nucleotide polymorphism (SNP) and/or a species-specific oligonucleotide polymorphism (OP) indicative of only one of the species *M. chitwoodi, M. fallax, M. minor, M. naasi* or *M. hapla.*

In a preferred embodiment of such a method,
(a) the presence of *M. chitwoodi* is indicated by the presence of a species-specific SNP in the LSU rRNA gene or its transcription product selected from the group consisting of:
   - SNP: 417T of SEQ ID NO: 16;
   - SNP: 557T of SEQ ID NO: 17;
   - SNP: 655A of SEQ ID NO: 18;
   - SNP: 806A of SEQ ID NO: 19;
(b) the presence of *M. fallax* is indicated by the presence of a species-specific OP in the LSU rRNA gene or its transcription product selected from the group consisting of:
   - OP: 647T, 655G of SEQ ID NO: 20; and
   - OP: 890T, 895A, 899G, 900A, 901A of SEQ ID NO: 21;
(c) the presence of *M. minor* is indicated by the presence of a species-specific SNP and/or a species-specific OP in the LSU rRNA gene or its transcription product selected from the group consisting of:
   - SNP: 214C of SEQ ID NO: 22;
   - SNPs: 420C; 437T and 466G of SEQ ID NO: 23;
   - OP: 539T, 540C of SEQ ID NO: 24;
   - SNP: 577G of SEQ ID NO: 24;
   - OP: 579G, 580G of SEQ ID NO: 24;
   - SNPs: 614T and 640G of SEQ ID NO: 25; and
   - SNP: 693C of SEQ ID NO: 26;
(d) the presence of *M. naasi* is indicated by the presence of a species-specific SNP and/or a species-specific OP in the LSU rRNA gene or its transcription product selected from the group consisting of:
   - SNP: 78A of SEQ ID NO: 27;
   - SNP: 147A of SEQ ID NO: 28;
   - SNP: 274C of SEQ ID NO: 29;
   - SNPs: 399T and 422C of SEQ ID NO: 30;
   - OP: 464T, 465G, 466A of SEQ ID NO: 31;
   - OP: 630T, 633T, 634T of SEQ ID NO: 32; and
   - SNP: 640C of SEQ ID NO: 32;
   and/or
(e) the presence of *M. hapla* is indicated by the presence of a species-specific SNP and/or a species-specific OP in the LSU rRNA gene or its transcription product selected from the group consisting of:
   - SNPs: 76A; 86C and 126A of SEQ ID NO: 33;
   - OP: 80A, 81A of SEQ ID NO: 33;
   - SNP: 185G of SEQ ID NO: 34;
   - SNPs: 371A and 410A of SEQ ID NO: 35;
   - SNPs: 518A; and 534C of SEQ ID NO: 36;
   - OP: 540A, 541T, 542G of SEQ ID NO: 36; and
   - OP: 616C, 623C of SEQ ID NO: 37.

The additional testing of the sample may comprise for instance the steps of
(a) hybridizing an oligonucleotide comprising a nucleotide sequence complementary to at least a part of the LSU rRNA gene of a nematode, a transcription product thereof, or a complementary nucleic acid thereof under stringent conditions to said nucleic acid in said sample, wherein said oligonucleotide is complementary in the position of at least one species-specific polymorphism, e.g. as defined above, and
(b) detecting whether hybridization has occurred, for instance by detecting the formation of stable hybrids between said oligonucleotide and said nucleic acids in said sample, wherein hybridization of said oligonucleotide is indicative for the presence of said species of the meloidogyne nematode.

Again, in such methods of detecting species-specific sequences, the oligonucleotide may be detectably labelled. Preferably, the oligonucleotide is labelled with a reporter dye and a quenching dye.

In a highly preferred embodiment of the present invention the hybridization signal obtained for the positive detection of a member of a group of the meloidogyne nematode as based on detection of a polymorphism in the SSU rRNA gene is compared to the hybridization signal obtained for a positive detection of a particular species of the meloidogyne nematode as a member of said group as based on detection of a polymorphism in the LSU rRNA gene, wherein the presence of substantially equal hybridization signals is indicative of a positive confirmation of the presence of the species in the sample.

In another aspect, the present invention provides oligonucleotide as defined above, wherein said oligonucleotide is complementary in the position of at least one group-specific polymorphism and/or species-specific polymorphism as defined above, for instance as based on the SSU rRNA or LSU rRNA polymorphisms provided herein.

In yet another aspect, the present invention provides a kit of parts suitable for performing a method according to the present invention, comprising an oligonucleotide as described above and optionally one or more parts selected from the group consisting of nucleic acid extraction means, nucleic acid amplification means, nucleic acid hybridization means, nucleic acid ligation means and nucleic acid detection means.

### DESCRIPTION OF THE DRAWINGS

Figure 1 is a graphic presentation of the hybridization signal obtained during Real time PCR using LSU-based *M. minor-* specific primers as described in the Examples. 1: template M. minor (10⁻⁴); 2: template M. minor (10⁻⁵); 3: template M. minor (10⁻⁶); 4: template M. minor (10⁻⁷). *M. minor-*related non-targets (M. naasi 10⁻⁵, M. fallax 10⁻⁵, and M. hapla 10⁻⁵) and water control give no signal.
Figure 2 is a graphic presentation of the hybridization signal obtained during Real time PCR using LSU-based *M. naasi-* specific primers as described in the Examples. 1: template M. naasi (10⁻⁴); 2: template M. naasi (10⁻⁵); 3: template M. naasi (10⁻⁶); 4: template *M. naasi* (10⁻⁷). M. naasi -related non-targets (M. minor 10⁻⁵, M. fallax 10⁻⁵, and M. hapla 10⁻⁵) and water control give no signal.
Figure 3 is a graphic presentation of the hybridization signal obtained during Real time PCR using LSU-based *M. chitwoodi-* or *M. fallax*-specific primers as described in the Examples. 1: template *M. chitwoodi* (10⁻⁵) ; 2: template *M. fallax* (10⁻⁵); Related non-targets (*M*. *naasi, M. manor, M. hapla*) and water control give no signal.

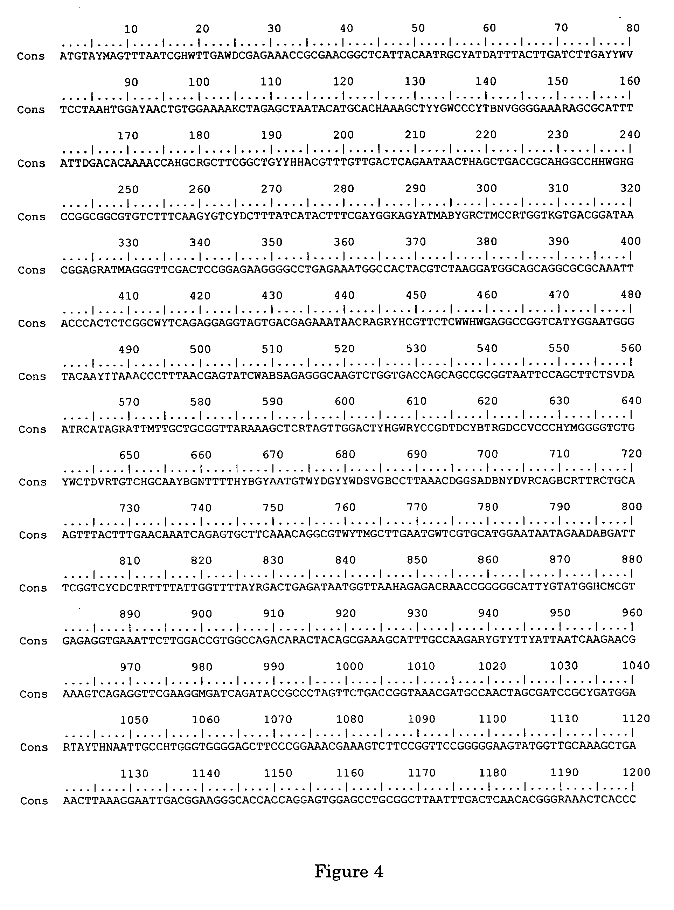

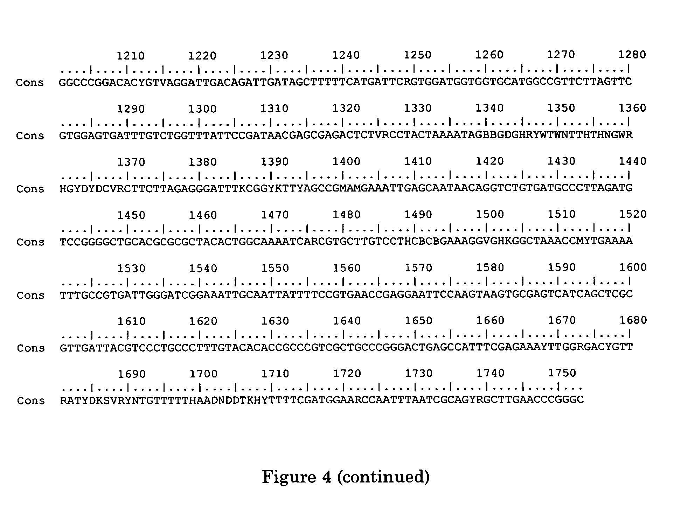

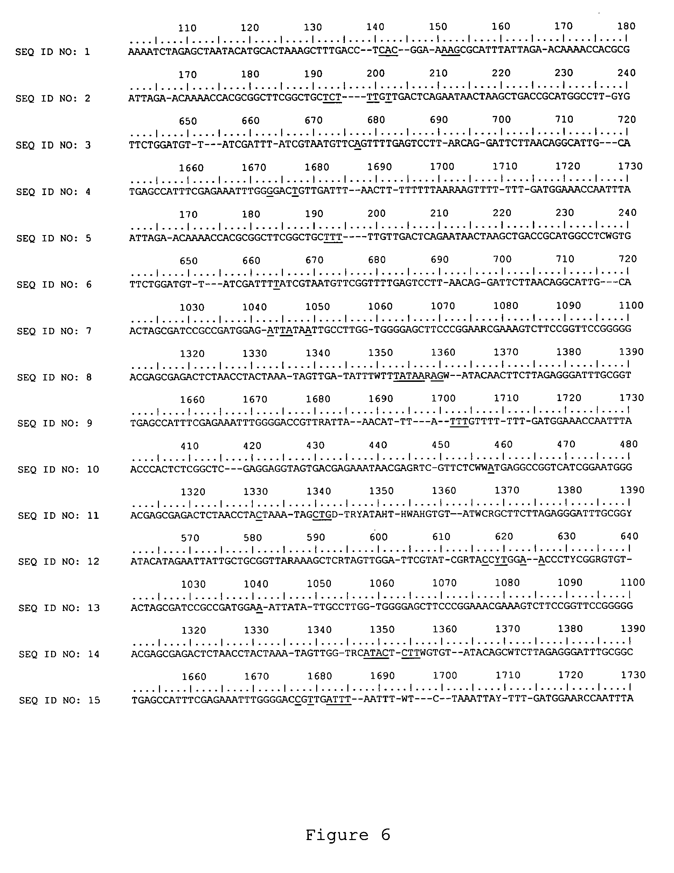

Figure 4 represents the consensus sequence of aligned SSU rDNA sequences obtained from a large number of meloidogyne nematodes and is provided to indicate the various nucleotide positions of SEQ ID NOs: 1-15 as provided in Figure 6 and to indicate the polymorphic positions of the SNPs and OPs listed in Table 2.

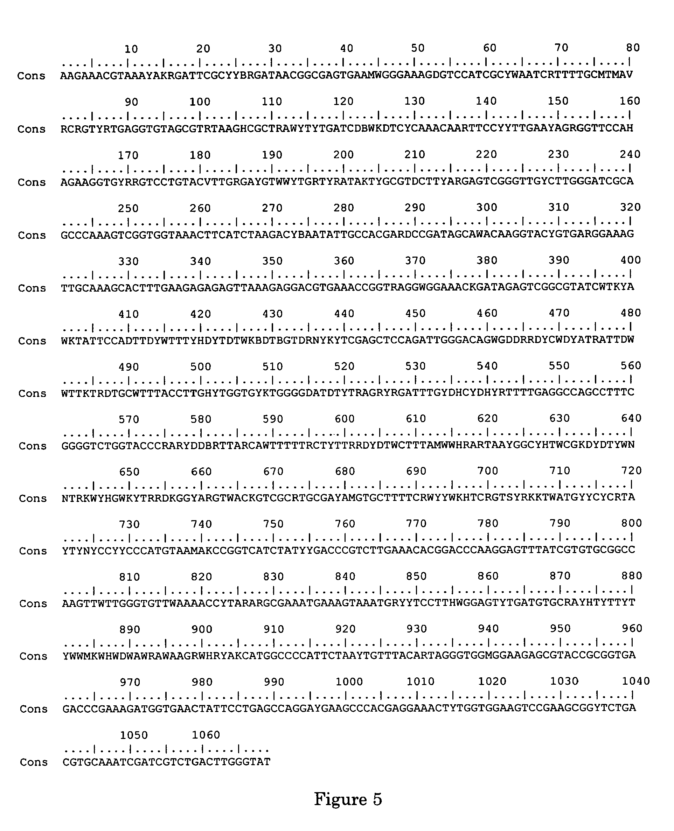

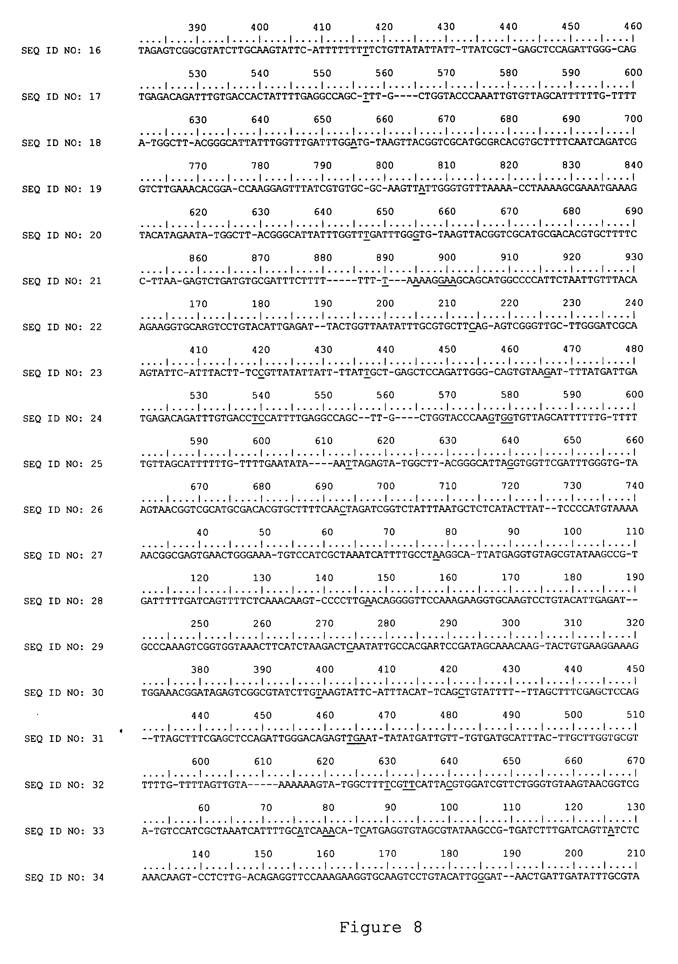

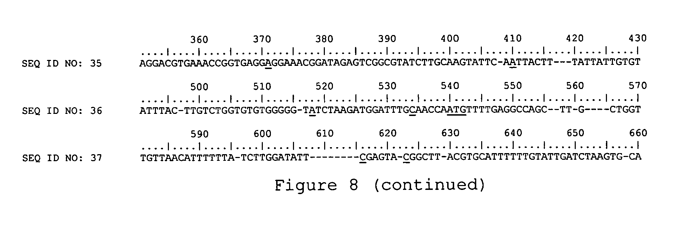

Figure 5 represents the consensus sequence of aligned LSU rDNA sequences obtained from a large number of meloidogyne nematodes and is provided to indicate the various nucleotide positions of SEQ ID NOs: 16-36 as provided in Figure 8 and to indicate the polymorphic positions of the SNPs and OPs of Table 3.
Figure 6 provides the nucleotide position of SEQ ID NOs:1-15 in the consensus alignment of Figure 4, wherein the sequences have introduced gaps for optimal alignment with the consensus sequence.

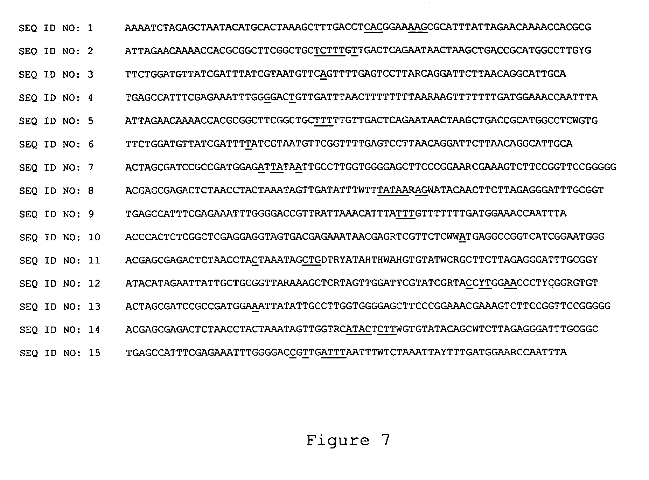

Figure 7 shows the SEQ ID NOs: 1-15 in plain text format without gaps for the groups A, B, C and D in their respective regions of the SSU rDNA comprising the SNPs or OPs as defined in Table 2.
Figure 8 provides the nucleotide position of SEQ ID NOs:16-37 in the consensus alignment of Figure 5, wherein the sequences have introduced gaps for optimal alignment with the consensus sequence.

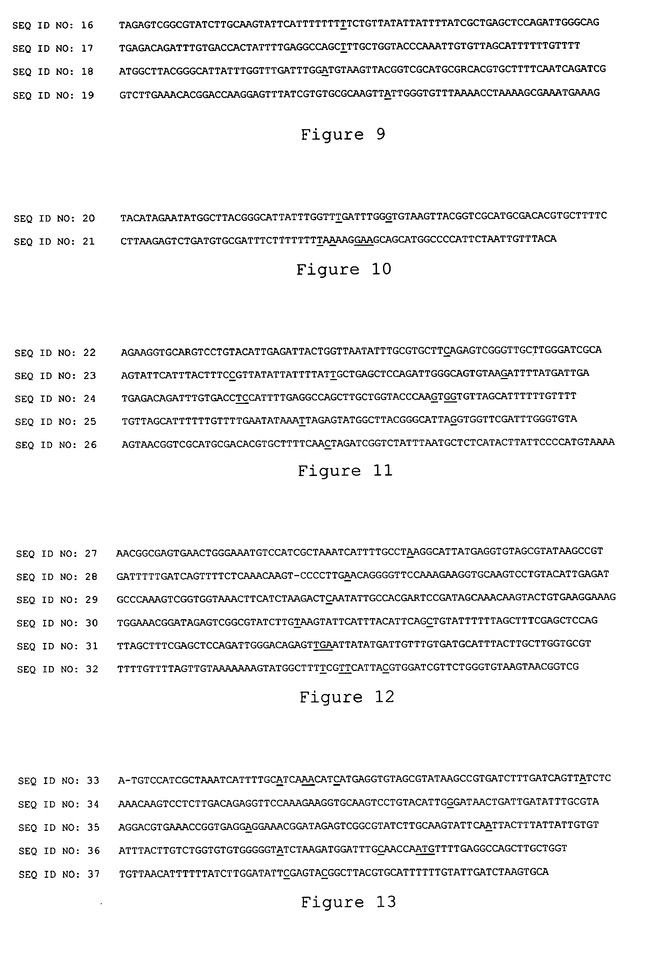

Figure 9 shows SEQ ID NOs: 16-19 in plain text format without gaps for *M. chitwoodi* in the regions of the LSU rDNA comprising the SNPs or OPs as defined in Table 3.
Figure 10 shows SEQ ID NOs: 20-21 in plain text format without gaps for *M. fallax* in the regions of the LSU rDNA comprising the SNPs or OPs as defined in Table 3.
Figure 11 shows SEQ ID NOs: 22-26 in plain text format without gaps for *M. minor* in the regions of the LSU rDNA comprising the SNPs or OPs as defined in Table 3.
Figure 12 shows the SEQ ID NOs: 27-32 in plain text format without gaps for *M. naasi* in the regions of the LSU rDNA comprising the SNPs or OPs as defined in Table 3.
Figure 13 shows the SEQ ID NOs: 33-37 in plain text format without gaps for *M. hapla* in the regions of the LSU rDNA comprising the SNPs or OPs as defined in Table 3.

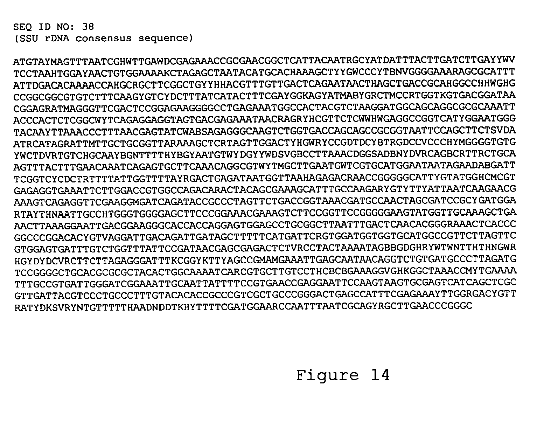

Figure 14 shows SEQ ID NO: 38 (consensus sequence of aligned SSU rDNA sequences) in plain text format without the nucleotide position numbering of Figure 4.

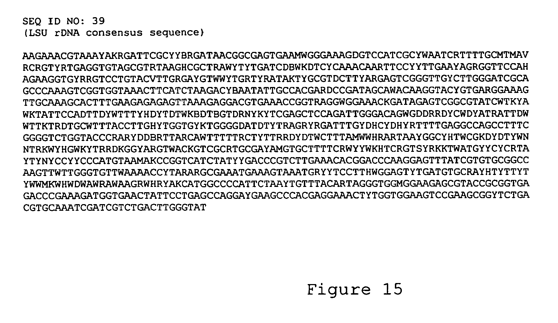

Figure 15 shows SEQ ID NO: 39 (consensus sequence of aligned LSU rDNA sequences) in plain text format without the nucleotide position numbering of Figure 5.

### DETAILED DESCRIPTION OF THE INVENTION

Nucleotides are referred to by their commonly accepted single-letter codes following IUPAC nomenclature: A (Adenine), C (Cytosine), T (Thymine), G (Guanine), U (Uracil), W (A or T), R (A or G), K (G or T), Y (C or T), S (C or G), M (A or C), B (C, G or T), H (A, C, or T), D (A, G, or T), V (A, C, or G), N (A, C, G, or T).

A "sample" as used herein refers specifically to an agricultural commodity, in particular a crop, more specifically a root or tuber, which is to be analyzed for the presence of RKN pests. The sample may thus suitably be the root or tuber itself, a soil sample extracted from the site where the commodity was grown, a soil sample attached to the commodity itself. Also, the sample may be a nematode specimen isolated from the commodity or any of the above referred soil samples. Alternatively, the sample may be a soil sample not associated with a commodity but requiring the analysis of the present invention. For instance, it may be a sample of a soil in which a commodity is to be grown, or a sample of a soil that is to be transported and for which a clearance is required wherein a demonstration of the absence of RKN is required.

The term "meloidogyne nematode" refers to nematodes of the taxonomic genus *Meloidogyne,* part of the *Meloidogynidae* family. The term is synonymous to the term "root knot nematodes", abbreviated as "RKN". Species of meloidogyne nematode include but are not limited to *M. arabicida, M. ardenensis, M. arenaria, M. artiellia, M. baetica, M. chitwoodi, M. duytsi, M. enterolobii, M. ethiopica, M. exigua, M. fallax, M. floridae, M. floridensis, M. graminicola, M. graminis, M. hapla, M. haplanaria, M. ichinohei, M. incognita, M. javanica, M. konaensis, M. maritime, M. mayaguensis, M. microtyla, M. minor, M. morocciensis, M. naasi, M. oryzae, M. panyuensis, M. paranaensis, M. partityla, M. sasseri, M. thailandica, M. trifoliophila and M. ulmi.*

The term "economically important root knot nematodes refers in particular to the species *M. arercaria, M. chitwoodi, M. fallax, M. hapla, M. incognita, M. javarcica, M. minor,* and *M. naasi.*

The term "group" is defined herein as an arbitrary clustering of species of meloidogyne nematodes which have a common polymorphism, either in the form of an SNP or an OP, with no taxonomic relevance other than that they can collectively be detected via the detection of the SNP or OP in the target nucleic acid, *in casu* the SSU or LSU rDNA. If a taxonomic level is to be accorded to such a group, it is on a taxonomic level below that of the genus, but above that of the species.

The term "single nucleotide polymorphism", abbreviated "SNP", as used herein refers to a DNA sequence variation that involves a substitution, insertion or deletion, generally an alteration, of a single nucleotide position.

The term "oligonucleotide polymorphism", abbreviated "OP", as used herein refers to a DNA sequence variation that involves a substitution, insertion or deletion, generally an alteration, of multiple nucleotide positions within a window of about 5-30 nucleotides. Generally, only a few nucleotides within the window of an OP are polymorphic, but the window as a whole can suitably be used as a unique sequence motif to distinguish a meloidogyne nematode from other nematodes and to define a short region of an rRNA gene or its transcription product as unique to certain group or a certain species.

The terms "species-specific single nucleotide polymorphism" and the corresponding term "species-specific oligonucleotide polymorphism" refer to SNPs, respectively OPs, on the basis of which the meloidogyne species can be distinguished from other species of nematodes.

The terms "group-specific single nucleotide polymorphism" and the corresponding term "group-specific oligonucleotide polymorphism" refer to SNPs, respectively OPs, on the basis of which the group can be distinguished from other species of nematodes. Thus the term as used herein can be defined as a polymorphism that is present in a group of nematode species, in particular meloidogyne nematodes, which group includes at least one economically important species of RKN (i.e. *M. arenaria, M. chitwoodi, M. fallax, M. hapla, M. incognita, M. javanica, M. minor* and/or *M. naasi*), and that is absent in other nematodes. Group-specific SNPs may for instance be detected by sequence comparison between two or more rDNA sequences, wherein the two sequences are aligned for maximum correspondence, in the case of rDNA taking into account the molecule's secondary structure. When two rDNA sequences are optimally aligned, both may include additions or deletions (i.e. gaps) relative to each other for optimal alignment of the two sequences. Optimal alignment of sequences for comparison may be conducted by computerized implementations of known algorithms, or by inspection. Readily available sequence comparison and multiple sequence alignment algorithms are, respectively, the Basic Local Alignment Search Tool (BLAST) (Altschul, S.F. et al. 1990. J. Mol. Biol. 215:403; Altschul, S.F. et al. 1997. Nucleic Acid Res. 25:3389-3402) and ClustalW programs both available on the internet. Other suitable programs include GAP, BESTFIT and FASTA in the Wisconsin Genetics Software Package (Genetics Computer Group (GCG), Madison, WI, USA).

When the rDNA sequences of a large number of properly identified meloidogyne nematodes are aligned with each other, the alignment may or may not reveal the presence of nucleotide positions (or nucleotide variations) that are unique to a single species of meloidogyne nematode. If present, such positions are termed species-specific SNPs herein when that species is an economically important meloidogyne nematode.

When the rDNA sequences of a large number of properly identified meloidogyne nematodes are aligned with a large number of free-living nematodes, the chances of finding a unique nucleotide position for a single species of meloidogyne nematode is greatly reduced. However, such a species-specific SNP would be very suitable for direct detection of a single species in a soil sample. Thus, the larger the alignment (large as in number of species), the smaller the chance of detecting diagnostically relevant SNPs, yet the more reliable the test will be in soil.

It has now been found that group-specific SNPs can be discovered in an alignment of rDNA sequences of meloidogyne nematodes together with a large number of free-living nematodes, i.e. such an alignment reveals nucleotide positions that are unique to more than one species of meloidogyne nematodes, either relative to other meloidogyne nematodes, but more importantly relative to the free-living nematodes. What is of agricultural significance is the finding that such group-specific SNPs can be found for groups that comprise at least one economically important meloidogyne nematode.

Thus, an alignment of rDNA sequences of meloidogyne nematodes may not reveal a single agriculturally important SNP, whereas in the case that the alignment is extended with free-living species of nematodes, the alignment may reveal nucleotide positions unique to all meloidogyne nematodes. Such group-specific SNPs are also of interest for the detection of meloidogyne nematodes in soil.

Moreover, SNPs (either group- or species-specific) may be present in adjacent positions, or may be present in one or more positions over a length of 2 to about 30 consecutive positions, optionally interspersed by non-informative positions. Such an array of polymorphisms over a limited length of the sequence is termed an oligonucleotide polymorphism herein.

Thus a polymorphic position may be present in all meloidogyne nematode species, in only a single meloidogyne nematode species, or in several but not all meloidogyne nematode species. As long as the polymorphic position is (also) present in at least one species of the group consisting of *M. arenaria, M. chitwoodi, M. fallax, M. hapla, M. incognita, M. javanica, M. minor* and *M. naasi,* that nucleotide polymorphism is considered informative for the present purpose and may be used in a test according to the present invention, either alone, or in combination with other informative nucleotide polymorphisms.

The term "ribosomal RNA" refers to the transcription products of the nematode rDNA cistron, which consists of several hundred tandemly repeated copies of the transcribed units (small subunit or SSU or 18S; large subunit or LSU or 28S and 5.8S; internal and external transcribed spacers) and an external nontranscribed or intergenic spacer.

A "coding" or "encoding" sequence is the part of a gene that codes for the amino acid sequence of a protein, or for a functional RNA such as a tRNA or rRNA.

A "complement" or "complementary sequence" is a sequence of nucleotides which forms a hydrogen-bonded duplex with another sequence of nucleotides according to Watson-Crick base-paring rules. For example, the complementary base sequence for 5'-AAGGCT-3' is 3'-TTCCGA-5'.

As used herein, "substantially complementary" means that two nucleic acid sequences have at least about 65%, preferably about 70%, more preferably about 80%, even more preferably 90%, and most preferably about 98%, sequence complementarity to each other. This means that the primers and probes must exhibit sufficient complementarity to their template and target nucleic acid, respectively, to hybridise under stringent conditions. Therefore, the primer sequences as disclosed in this specification need not reflect the exact sequence of the binding region on the template and degenerate primers can be used. A substantially complementary primer sequence is one that has sufficient sequence complementarity to the amplification template to result in primer binding and second-strand synthesis.

The term "hybrid" refers to a double-stranded nucleic acid molecule, or duplex, formed by hydrogen bonding between complementary nucleotides. The terms "hybridise" or "anneal" refer to the process by which single strands of nucleic acid sequences form double-helical segments through hydrogen bonding between complementary nucleotides.

The term "oligonucleotide" refers to a short sequence of nucleotide monomers (usually 6 to 100 nucleotides) joined by phosphorous linkages (e.g., phosphodiester, alkyl and aryl-phosphate, phosphorothioate), or non-phosphorous linkages (e.g., peptide, sulfamate and others). An oligonucleotide may contain modified nucleotides having modified bases (e.g., 5-methyl cytosine) and modified sugar groups (e.g., 2'-O-methyl ribosyl, 2'-O-methoxyethyl ribosyl, 2'-fluoro ribosyl, 2'-amino ribosyl, and the like). Oligonucleotides may be naturally-occurring or synthetic molecules of double-and single-stranded DNA and double- and single-stranded RNA with circular, branched or linear shapes and optionally including domains capable of forming stable secondary structures (e.g., stem-and-loop and loop-stem-loop structures).

The term "primer" as used herein refers to an oligonucleotide which is capable of annealing to the amplification target allowing a DNA polymerase to attach thereby serving as a point of initiation of DNA synthesis when placed under conditions in which synthesis of primer extension product which is complementary to a nucleic acid strand is induced, i.e., in the presence of nucleotides and an agent for polymerization such as DNA polymerase and at a suitable temperature and pH. The (amplification) primer is preferably single stranded for maximum efficiency in amplification. Preferably, the primer is an oligodeoxy ribonucleotide. The primer must be sufficiently long to prime the synthesis of extension products in the presence of the agent for polymerization. The exact lengths of the primers will depend on many factors, including temperature and source of primer. A "pair of bi-directional primers" as used herein refers to one forward and one reverse primer as commonly used in the art of DNA amplification such as in PCR amplification.

The term "probe" refers to a single-stranded oligonucleotide sequence that will recognize and form a hydrogen-bonded duplex with a complementary sequence in a target nucleic acid sequence analyte or its cDNA derivative.

The terms "stringency" or "stringent hybridization conditions" refer to hybridization conditions that affect the stability of hybrids, e.g., temperature, salt concentration, pH, formamide concentration and the like. These conditions are empirically optimised to maximize specific binding and minimize nonspecific binding of primer or probe to its target nucleic acid sequence. The terms as used include reference to conditions under which a probe or primer will hybridise to its target sequence, to a detectably greater degree than other sequences (e.g. at least 2-fold over background). Stringent conditions are sequence dependent and will be different in different circumstances. Longer sequences hybridise specifically at higher temperatures. Generally, stringent conditions are selected to be about 5°C lower than the thermal melting point (Tₘ) for the specific sequence at a defined ionic strength and pH. The Tₘ is the temperature (under defined ionic strength and pH) at which 50% of a complementary target sequence hybridises to a perfectly matched probe or primer. Typically, stringent conditions will be those in which the salt concentration is less than about 1.0 M Na⁺ ion, typically about 0.01 to 1.0 M Na⁺ ion concentration (or other salts) at pH 7.0 to 8.3 and the temperature is at least about 30°C for short probes or primers (e.g. 10 to 50 nucleotides) and at least about 60°C for long probes or primers (e.g. greater than 50 nucleotides). Stringent conditions may also be achieved with the addition of destabilizing agents such as formamide. Exemplary low stringent conditions or "conditions of reduced stringency" include hybridization with a buffer solution of 30% formamide, 1 M NaCl, 1% SDS at 37°C and a wash in 2x SSC at 40°C. Exemplary high stringency conditions include hybridization in 50% formamide, 1 M NaCl, 1% SDS at 37°C, and a wash in 0.1x SSC at 60°C. Hybridization procedures are well known in the art and are described in e.g. Ausubel, F.M., Brent, R., Kingston, R.E., Moore, D.D.,Seidman, J.G., Smith, J.A., Struhl, K. eds. (1998) Current protocols in molecular biology. V.B. Chanda, series ed. New York: John Wiley & Sons.

Methods of the invention can in principle be performed by using any nucleic acid amplification method, such as the Polymerase Chain Reaction (PCR; Mullis 1987, U.S. Pat. No. 4,683,195, 4,683,202, en 4,800,159) or by using amplification reactions such as Ligase Chain Reaction (LCR; Barany 1991, Proc. Natl. Acad. Sci. USA 88:189-193; EP Appl. No., 320,308), Self-Sustained Sequence Replication (3SR; Guatelli et al., 1990, Proc. Natl. Acad. Sci. USA 87:1874-1878), Strand Displacement Amplification (SDA; U.S. Pat. Nos. 5,270,184, en 5,455,166), Transcriptional Amplification System (TAS; Kwoh et al., Proc. Natl. Acad. Sci. USA 86:1173-1177), Q-Beta Replicase (Lizardi et al., 1988, Bio/Technology 6:1197), Rolling Circle Amplification (RCA; U.S. Pat. No. 5,871,921), Nucleic Acid Sequence Based Amplification (NASBA), Cleavase Fragment Length Polymorphism (U.S. Pat. No. 5,719,028), Isothermal and Chimeric Primer-initiated Amplification of Nucleic Acid (ICAN), Ramification-extension Amplification Method (RAM; U.S. Pat. Nos. 5,719,028 and 5,942,391) or other suitable methods for amplification of DNA.

In order to amplify DNA with a small number of mismatches to one or more of the amplification primers, an amplification reaction may be performed under conditions of reduced stringency (e.g. a PCR amplification using an annealing temperature of 38°C, or the presence of 3.5 mM MgCl₂). The person skilled in the art will be able to select conditions of suitable stringency.

The primers herein are selected to be "substantially" complementary (i.e. at least 65%, more preferably at least 80% perfectly complementary) to their target regions present on the different strands of each specific sequence to be amplified. It is possible to use primer sequences containing e.g. inositol residues or ambiguous bases or even primers that contain one or more mismatches when compared to the target sequence. In general, sequences that exhibit at least 65%, more preferably at least 80% homology with the target DNA oligonucleotide sequences, are considered suitable for use in a method of the present invention. Sequence mismatches are also not critical when using low stringency hybridization conditions.

The detection of the amplification products can in principle be accomplished by any suitable method known in the art. The detection fragments may be directly stained or labelled with radioactive labels, antibodies, luminescent dyes, fluorescent dyes, or enzyme reagents. Direct DNA stains include for example intercalating dyes such as acridine orange, ethidium bromide, ethidium monoazide or Hoechst dyes.

Alternatively, the DNA fragments may be detected by incorporation of labelled dNTP bases into the synthesized DNA fragments. Detection labels which may be associated with nucleotide bases include e.g. fluorescein, cyanine dye or BrdUrd.

When using a probe-based detection system, a suitable detection procedure for use in the present invention may for example comprise an enzyme immunoassay (EIA) format (Jacobs et al., 1997, J. Clin. Microbiol. 35, 791-795). For performing a detection by manner of the EIA procedure, either the forward or the reverse primer used in the amplification reaction may comprise a capturing group, such as a biotin group for immobilization of target DNA PCR amplicons on e.g. a streptavidin coated microtiter plate wells for subsequent EIA detection of target DNA -amplicons (see below). The skilled person will understand that other groups for immobilization of target DNA PCR amplicons in an EIA format may be employed.

Probes useful for the detection of the target DNA as disclosed herein preferably bind only to at least a part of the DNA sequence region as amplified by the DNA amplification procedure. Those of skill in the art can prepare suitable probes for detection based on the nucleotide sequence of the target DNA without undue experimentation as set out herein. Also the complementary sequences of the target DNA may suitably be used as detection probes in a method of the invention, provided that such a complementary strand is amplified in the amplification reaction employed.

Suitable detection procedures for use herein may for example comprise immobilization of the amplicons and probing the DNA sequences thereof by e.g. southern blotting. Other formats may comprise an EIA format as described above. To facilitate the detection of binding, the specific amplicon detection probes may comprise a label moiety such as a fluorophore, a chromophore, an enzyme or a radio-label, so as to facilitate monitoring of binding of the probes to the reaction product of the amplification reaction. Such labels are well-known to those skilled in the art and include, for example, fluorescein isothiocyanate (FITC), β-galactosidase, horseradish peroxidase, streptavidin, biotin, digoxigenin, ³⁵S or ¹²⁵I. Other examples will be apparent to those skilled in the art.

Detection may also be performed by a so called reverse line blot (RLB) assay, such as for instance described by Van den Brule et al. (2002, J. Clin. Microbiol. 40, 779-787). For this purpose RLB probes are preferably synthesized with a 5'amino group for subsequent immobilization on e.g. carboxyl-coated nylon membranes. The advantage of an RLB format is the ease of the system and its speed, thus allowing for high throughput sample processing.

Any suitable method for screening the nucleic acids for the presence or absence of polymorphisms is considered to be part of the instant invention. Such methods include, but are not limited to: DNA sequencing, restriction fragment length polymorphism (RFLP) analysis, amplified fragment length polymorphism (AFLP) analysis; heteroduplex analysis, single strand conformational polymorphism (SSCP) analysis, denaturing gradient gel electrophoresis (DGGE), real time PCR analysis (e.g. Taqman®), temperature gradient gel electrophoresis (TGGE), primer extension, allele-specific hybridization, and INVADER® genetic analysis assays, cleavase fragment length polymorphism (CFLP) analysis, sequence-characterized amplified region (SCAR) analysis, cleaved amplified polymorphic sequence (CAPS) analysis

The use of nucleic acid probes for the detection of specific DNA sequences is well known in the art. Mostly these procedures comprise the hybridization of the target DNA with the probe followed by post-hybridization washings. Specificity is typically the function of post-hybridization washes, the critical factors being the ionic strength and temperature of the final wash solution. For DNA-DNA hybrids, the Tm can be approximated from the equation of Meinkoth and Wahl, Anal. Biochem., 138: 267-284 (1984): Tm = 81.5 °C + 16.6 (log M) + 0.41 (% GC)-0.61 (% form)-500/L; where M is the molarity of monovalent cations, % GC is the percentage of guanosine and cytosine nucleotides in the DNA, % form is the percentage of formamide in the hybridization solution, and L is the length of the hybrid in base pairs. The Tm is the temperature (under defined ionic strength and pH) at which 50% of a complementary target sequence hybridizes to a perfectly matched probe. Tm is reduced by about 1 °C for each 1 % of mismatching; thus, the hybridization and/or wash conditions can be adjusted to hybridize to sequences of the desired identity. For example, if sequences with > 90% identity are sought, the Tm can be decreased 10 C. Generally, stringent conditions are selected to be about 5 C lower than the thermal melting point (Tm) for the specific sequence and its complement at a defined ionic strength and pH. However, severely stringent conditions can utilize a hybridization and/or wash at 1,2,3, or 4 °C lower than the thermal melting point (Tm); moderately stringent conditions can utilize a hybridization and/or wash at 6,7,8,9, or 10 °C lower than the thermal melting point (Tm); low stringency conditions can utilize a hybridization and/or wash at 11,12,13,14,15, or 20 °C lower than the thermal melting point (Tm). Using the equation, hybridization and wash compositions, and desired Tm, those of ordinary skill will understand that variations in the stringency of hybridization and/or wash solutions are inherently described. If the desired degree of mismatching results in a Tm of less than 45 °C (aqueous solution) or 32 °C (formamide solution) it is preferred to increase the SSC concentration so that a higher temperature can be used. An extensive guide to the hybridization of nucleic acids is found in Tijssen, Laboratory Techniques in Biochemistry and Molecular Biology-Hybridization with Nucleic Acid Probes, Part I, Chapter 2 "Overview of principles of hybridization and the strategy of nucleic acid probe assays", Elsevier. New York (1993); and Current Protocols in Molecular Biology, Chapter 2, Ausubel, et al., Eds., *supra.*

It is allowable for detection probes of the present invention to contain one or more mismatches to their target sequence. In general, sequences that exhibit at least 65%, more preferably at least 80% homology with the target DNA oligonucleotide sequences are considered suitable for use in a method of the present invention.

It is known that single nucleotide polymorphisms (SNPs) in ribosomal RNA genes may in certain applications prove useful for the detection of nematodes. EP 1 613 772, for instance, describes a method for determining soil health, wherein SNPs of ribosomal RNA genes are proposed for the detection of nematodes. However, EP 1 613 772 is aimed at describing the total soil community, particularly the free-living part, and provides no solution for the above-described problems of detecting RKN in samples.

Moreover, in view of the fact that the ribosomal RNA genes represent coding sequences with evolutionary pressure towards conservation of the tertiary (and thus primary) structure, it is unlikely that the ribosomal RNA genes may provide any basis for a reliable detection system.

This was confirmed by a comprehensive study by de Ley et al. (Journal of Nematology, 2002, Vol. 34(4):319-327) into the phylogenetic relationship between meloidogyne nematodes wherein it was concluded that potential nucleotide polymorphisms in the meloidogyne SSU rDNA sequences are rare to absent, and that only a number of species of lesser economic significance are sufficiently divergent to contemplate diagnostic applications based on SSU rDNA sequence data.

It is the more surprising that the present inventors were able to device a test based on rRNA data that is reliable enough to even perform analysis in crude soil samples, wherein not only meloidogyne nematodes reside, but thousands of other nematode species.

The present invention now provides various examples of suitable group-specific single nucleotide polymorphism (SNP) and/or a group-specific oligonucleotide polymorphism (OP), which may be used for the detection of groups of meloidogyne nematodes of interest. The one of average skill in the art will appreciate that other suitable polymorphisms may be discovered that can be used in methods of the present invention. As described in the section on the meaning of the terms "group-specific single nucleotide polymorphism" and the corresponding term "group-specific oligonucleotide polymorphism" herein above, the skilled person is provided with sufficient teaching to find additional polymorphisms among aligned rRNA sequences of nematodes. It is within the realm of average skill to obtain rRNA gene sequence data from isolated and taxonomically classified nematodes and to align the sequences thus obtained.

Also, the development of primers and probes useful for the detection of polymorphic positions in a nucleic acid is within the realm of ordinary skill (see for instance Sambrook, J., Russell D.W., Sambrook, J. (2001) Molecular Cloning: a Laboratory Manual. Cold Spring Harbor Laboratory Press, Plainview, N.Y.).

By using standard DNA technology it is possible to produce probes and primers that directly or indirectly hybridize to the RNA or DNA of the meloidogyne nematodes (or their complement), or cDNA produced there from by reverse transcription, and which can be used in assays for the detection of the nematodes. Nucleic acid amplification techniques allow the amplification of fragments of nematode nucleic acids, which may be present in very low amounts.

In order to develop nucleic acid-based detection methods, nematodes-specific sequences must be determined for which primers or probes may then be developed. To detect the meloidogyne nematodes by nucleic acid amplification and/or probe hybridization, the rRNA genes or ribosomal RNA may be isolated from purified meloidogyne nematodes, optionally reverse transcribed into cDNA and directly cloned and/or sequenced. Using either the cloned nucleic acid as a hybridization probe, using sequence information derived from the clone, or by designing degenerative primers based on the sequence of the rRNA gene, nucleic acid hybridization probes and/or nucleic acid amplification primers may be designed an used in a detection assay for detecting the presence of the meloidogyne nematode in a sample as defined herein.

Since the meloidogyne nematode comprises both DNA and rRNA, a suitable detection method may comprise isolating the meloidogyne nematode nucleic acids from a sample, for instance from a plant root, by using methods known *per se* to the skilled person. DNA and RNA isolation kits are commercially available from for instance QIAGEN GmbH, Hilden, Germany, or Roche Diagnostics, a division of F. Hoffmann-La Roche Ltd, Basel, Switzerland.

Total RNA may for instance be extracted from a meloidogyne nematode, a plant root material or a soil sample, and the total RNA, or specifically the meloidogyne nematode ribosomal RNA, or a part thereof, may then be reverse transcribed into cDNA by using for instance an *Avian myeloblastosis virus* (AMV) reverse transcriptase or *Moloney murine leukemia virus* (M-MuLV) reverse transcriptase. A suitable method may for instance include mixing into a suitable aqueous buffering system (e.g. a commercially available RT buffer) a suitable amount of total RNAs (e.g. 1 to 5 µg), a suitable amount (e.g. 10 pmol) of a reverse transcription primer, a suitable amount of dNTPs and the reverse transcriptase, denaturing the nucleic acids by boiling for 1 min, and chilling them on ice, followed by reverse transcription at for instance 45°C for 1 h as recommended for the specific reverse transcriptase used, to obtain cDNA copies of the RNA sequences.

As a reverse transcription primer a polynucleotide according to the present invention may be used, for instance an 18-25-mer oligonucleotide comprising a nucleotide sequence complementary to the meloidogyne nematode rRNA sequence, such as a universally conserved rRNA sequence, or a polymorphic position as described herein.

Following the RT-step, the cDNA obtained may be PCR amplified by using for instance Pfu and Taq DNA polymerases and amplification primers specific for the meloidogyne nematode cDNA sequences. Also complete commercially available systems may be used for RT-PCR (e.g. the Access and AccessQuick™ RT-PCR Systems of Promega [Madison WI, USA], or the Titan™ One Tube RT-PCR System or two-step RT-PCR systems provided by Roche Diagnostics [a division of F. Hoffmann-La Roche Ltd, Basel, Switzerland]).

Alternatively, total DNA may for instance be extracted from a meloidogyne nematode, a plant root material or a soil sample, and the total RNA, or specifically the meloidogyne nematode ribosomal RNA gene, or a part thereof, may be PCR amplified by using for instance Pfu and Taq DNA polymerases and amplification primers specific for the meloidogyne nematode rDNA sequences. Also complete commercially available systems may be used for PCR (e.g. available form various suppliers such as Roche Diagnostics). A suitable method may for instance include mixing into a suitable aqueous buffering system (e.g. a commercially available PCR buffer) a suitable amount of total DNA as a template (e.g. 1 to 5 µg), a suitable amount (e.g. 10 pmol) of a pair of bi-directional amplification primers, a suitable amount of dNTPs and the DNA polymerase, denaturing the nucleic acids by boiling for 1 min, and performing a cycling reaction of around 10-50 alternating cycles of stringent primer hybridization, strand elongation and denaturing, at suitable temperatures to obtain DNA copies of the DNA template as amplification product. The amount of copies produced upon a certain number of cycles correlates directly to the amount of target DNA in the DNA template.

The skilled person is well aware of the available quantitative PCR methods presently available from commercial suppliers to quantify the amount of target DNA in the template. The term "hybridization signal" as used herein *inter alia* refers to the amount of amplification product produced upon a certain number of cycles and thus to the amount of target DNA available as template in the reaction. Thus, at equal hybridization signals between group-specific and species-specific tests as described herein, the amount of target template DNA in the sample is equal for both tests, indicating that a single species was detected in both tests.

In order to amplify a nucleic acid with a small number of mismatches to one or more of the amplification primers, an amplification reaction may be performed under conditions of reduced stringency (e.g. a PCR amplification using an annealing temperature of 38°C, or the presence of 3.5 mM MgCl₂). The person skilled in the art will be able to select conditions of suitable stringency.

The primers herein are selected to be "substantially" complementary (*i*.*e*. at least 65%, more preferably at least 80% perfectly complementary) to their target regions present on the different strands of each specific sequence to be amplified. It is possible to use primer sequences containing e.g. inositol residues or ambiguous bases or even primers that contain one or more mismatches when compared to the target sequence. In general, sequences that exhibit at least 65%, more preferably at least 80% homology with the target DNA or RNA oligonucleotide sequences, are considered suitable for use in a method of the present invention. Sequence mismatches are also not critical when using low stringency hybridization conditions.

The detection of the amplification products can in principle be accomplished by any suitable method known in the art. The amplified fragments may be directly stained or labelled with radioactive labels, antibodies, luminescent dyes, fluorescent dyes, or enzyme reagents. Direct DNA stains include for example intercalating dyes such as acridine orange, ethidium bromide, ethidium monoazide or Hoechst dyes.

Alternatively, the DNA or RNA fragments may be detected by incorporation of labelled dNTP bases into the synthesized fragments. Detection labels which may be associated with nucleotide bases include e.g. fluorescein, cyanine dye, digoxigenin (DIG) or bromodeoxyuridine (BrdUrd).

In a quantitative PCR method, the reaction is preferably performed by using an oligonucleotide primer that contains one or more 'locked' nucleic acid (LNA®) monomers, or by using LNA® fluorescent probes. LNA® technology involves an oligonucleotide (probe or primer that contains one or more LNA® monomers [2'-*O*, 4'-*C*-methylene-β-D-ribofuranosyl-modified] (e.g. Petersen and Wengel, 2003. TRENDS in Biotechnology Vol.21(2):74-81). In an LNA monomer, the ribose sugar moiety of the nucleotide is modified, while the base itself is unaltered. The result is a covalent bridge that 'locks' the ribose in the N-type (3'-endo) conformation, which enhances base stacking and phosphate backbone pre-organisation. This provides the oligonucleotide with improved affinity for complementary DNA or RNA sequences and therefore a higher *T*ₘ. When using LNA® primers, the detection of the double stranded amplification products may for instance be performed by using a double-stranded DNA stain, such as SYBR Green® [Molecular Probes, Inc.] (see for instance Ponchel et al. 2003. BMC Biotechnology 3:18).

Other methods of analysing the nuclei acid suitably comprise the use of a primer extension assay; a Taqman® PCR; a differential hybridization assay; an assay which detects allele-specific enzyme cleavage; and/or allele-specific PCR.

When using a probe-based detection system, a suitable detection procedure for use in the present invention may for example comprise an enzyme immunoassay (EIA) format (Jacobs et al., 1997. J Clin Microbiol 35:791-795). For performing a detection by manner of the EIA procedure, either the forward or the reverse primer used in the amplification reaction may comprise a capturing group, such as a biotin group for immobilization of target DNA PCR amplicons on e.g. a streptavidin coated microtiter plate wells or streptavidin coated Dynabeads® (Dynal Biotech, Oslo, Norway) for subsequent EIA detection of target DNA-amplicons. The skilled person will understand that other groups for immobilization of target DNA PCR amplicons in an EIA format may be employed.

Probes useful for the detection of the target nucleic acid sequences as disclosed herein preferably bind only to at least a part of the nucleic acid sequence region as amplified by the nucleic acid amplification procedure. Those of skill in the art can prepare suitable probes for detection based on the nucleotide sequence of the target nucleic acid without undue experimentation as set out herein. Also the complementary nucleotide sequences, whether DNA or RNA or chemically synthesized analogues, of the target nucleic acid may suitably be used as type-specific detection probes in a method of the invention, provided that such a complementary strand is amplified in the amplification reaction employed.

Suitable detection procedures for use herein may for example comprise immobilization of the amplicons and probing the nucleic acid sequences thereof by e.g. Northern and Southern blotting. Other formats may comprise an EIA format as described above. To facilitate the detection of binding, the specific amplicon detection probes may comprise a label moiety such as a fluorophore, a chromophore, an enzyme or a radio-label, so as to facilitate monitoring of binding of the probes to the reaction product of the amplification reaction. Such labels are well known to those skilled in the art and include, for example, fluorescein isothiocyanate (FITC), β-galactosidase, horseradish peroxidase, streptavidin, biotin, digoxigenin, ³⁵S, 14C, 32P or ¹²⁵I. Other examples will be apparent to those skilled in the art.

Detection may also be performed by a so-called reverse line blot (RLB) assay, such as for instance described by Van den Brule *et al.* (2002). For this purpose RLB probes are preferably synthesized with a 5' amino group for subsequent immobilization on e.g. carboxyl-coated nylon membranes. The advantage of an RLB format is the ease of the system and its speed, thus allowing for high throughput sample processing.

The use of nucleic acid probes for the detection of RNA or DNA fragments is well known in the art. Mostly these procedures comprise the hybridization of the target nucleic acid with the probe followed by post-hybridization washings. Specificity is typically the function of post-hybridization washes, the critical factors being the ionic strength and temperature of the final wash solution. For nucleic acid hybrids, the Tm can be approximated from the equation of Meinkoth and Wahl (1984): Tm = 81.5 °C + 16.6 (log M) + 0.41 (% GC)-0.61 (% form)-500/L; where M is the molarity of monovalent cations, % GC is the percentage of guanosine and cytosine nucleotides in the nucleic acid, % form is the percentage of formamide in the hybridization solution, and L is the length of the hybrid in base pairs. The Tm is the temperature (under defined ionic strength and pH) at which 50% of a complementary target sequence hybridizes to a perfectly matched probe. Tm is reduced by about 1 °C for each 1 % of mismatching; thus, the hybridization and/or wash conditions can be adjusted to hybridize to sequences of the desired identity. For example, if sequences with > 90% identity are sought, the Tm can be decreased 10°C. Generally, stringent conditions are selected to be about 5 °C lower than the thermal melting point (Tm) for the specific sequence and its complement at a defined ionic strength and pH. However, severely stringent conditions can utilize a hybridization and/or wash at 1,2,3, or 4 °C lower than the thermal melting point (Tm); moderately stringent conditions can utilize a hybridization and/or wash at 6, 7, 8, 9, or 10 °C lower than the thermal melting point (Tm); low stringency conditions can utilize a hybridization and/or wash at 11, 12, 13, 14, 15, or 20 °C lower than the thermal melting point (Tm). Using the equation, hybridization and wash compositions, and desired Tm, those of ordinary skill will understand that variations in the stringency of hybridization and/or wash solutions are inherently described. If the desired degree of mismatching results in a Tm of less than 45 °C (aqueous solution) or 32 °C (formamide solution) it is preferred to increase the SSC concentration so that a higher temperature can be used. An extensive guide to the hybridization of nucleic acids is found in Tijssen, 1993 *supra*; Ausubel *et al.,* 1998 *supra.*

In another aspect, the invention provides oligonucleotide probes for the detection of meloidogyne nematode RNA or DNA. The detection probes herein are selected to be "substantially" complementary to a single stranded RNA molecule, or to one of the strands of the double stranded nucleic acids generated by an amplification reaction of the invention. Preferably the probes are substantially complementary to the, optionally immobilized (e.g. biotin labelled) antisense strands of the amplicons generated from the target RNA or DNA.

It is allowable for detection probes of the present invention to contain one or more mismatches to their target sequence. In general, sequences that exhibit at least 65%, more preferably at least 80% homology with the target oligonucleotide sequences are considered suitable for use in a method of the present invention.

The invention will now be illustrated by way of the following, non limiting Example.

### EXAMPLE

### Detection of polymorphisms and design of primers.

The procedure for preparation of the rDNA sequence alignments was essentially performed as described in WO2004/090164. For this, a large number (>300) of meloidogyne nematodes and free living nematodes was correctly identified using standard protocols based on morphological characteristics. In certain cases, the identification was confirmed by isozyme analysis. Upon correct identification, the DNA was isolated from a single nematode worm and the complete SSU rDNA sequence was determined for all nematodes available. This resulted in a massive alignment of SSU rDNA sequences. From this alignment, groups were formed (see Table 1) and group-specific SNPs and OPs were identified (see Table 2) as described in detail in the above description.

In the same way, an alignment of part of the LSU rDNA sequence was produced for a limited number of meloidogyne nematodes, and species-specific SNPs and OPs were identified (see Table 3).

Based on this information amplification primers were developed (see Table 4).

**Table 1. Distribution of Meloidogyne species in and outside Northwestern Europe.**

| **Group** | **Species name** (economically important species in bold) | **Northwestern (NW) Europe** | **Outside NW Europe** |
|---|---|---|---|
| A | ***M. fallax*** | + (mainly NL, B, D, F) | South Africa, New Zealand |
| | ***M. minor*** | + (mainly NL, UK, IE | - |
| | ***M. chitwoodi*** | + (mainly NL and B) | USA, Argentina, South Africa |
| | | | |
| B | ***M. naasi*** | + | USA, Chili, New Zealand |
| | *M. oryzae* | - | Surinam |
| | *M. graminicola* | - | Mainly South-East Asia |
| | | | |
| C | ***M. hapla*** | + (widely distributed) | (widely distributed) |
| | *M. microtyla* | - | Canada |
| | *M. ardenensis* | + (GB, G and NL) | |
| | *M. maritime* | + (mainly costal foredunes) | South Africa |
| | *M. duytsi* | + (mainly costal foredunes) | South Africa |
| | | | |
| D | ***M. incognita*** | - (except for greenhouses) | widely distributed |
| | ***M. arenaria*** | - (except for greenhouses) | widely distributed |
| | ***M. javanica*** | - (except for greenhouses) | widely distributed |

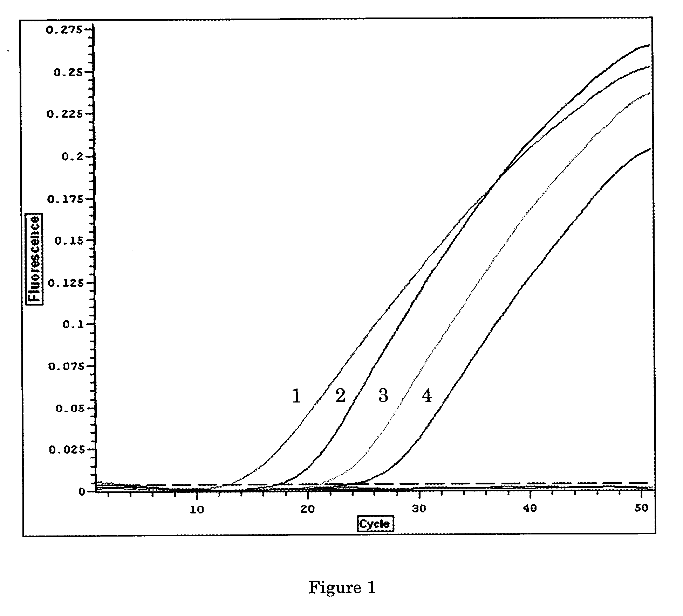

**Table 2 - SSU ribosomal DNA-based Meloidogyne group identification. Nucleotide positions are defined in Fig. 1. Motives (referred to herein as SNPs or OPs) were selected on the basis of all available SSU rDNA sequences from nematodes in the database of the Laboratory for Nematology, Wageningen, The Netherlands. The motives defined here are unique for the relevant group of Meloidogyne species.**

| **Group*** | **Group-specific (combinations of) nucleotides** | | | | | | |
|---|---|---|---|---|---|---|---|
| A | 141C, 142A, 143C | 192T, 193C, 194T | 677A | 1673G, 1677T | | | |
| | 151A, 152A, 153G | 199T, 200T, 202T | | | | | |
| B | 192T, 193T, 194T | 664T (3) | 1043A, 1045T, 1046A, | 1353T, 1354A, | 1359A, | 1702T, 1703T, | |
| | | | 1049A (3) | 1355T, | 1360 R (G) | 1704T (5) | |
| | | | | 1356A, 1357A (4) | (5) | | |
| C | 458A | 1331C, 1340C, | | | | | |
| | | 1341T, 1342G | | | | | |
| D | 617C, 620Y(T), 623A, 626A (1) | | 1348A, 1349T, 1350A, 1351C | | 1677C, 1679T, 1682A, | | 1041A |
| | | | 1354C, 1355T, 1356T (2) | | 1683T, 1684T, 1685T | | |

| | | | | | | | |
|---|---|---|---|---|---|---|---|
| (1) Cross reactivity with *M. maritime* - only found in coastal foredunes, may not include some populations of *M. ethiopica* and *M. javanica* (2) Cross reactivity with some *M. exigua* populations (3) includes *M. exigua* (not present in NW Europe) (4) does not include *M. graminicola* (not present in NW Europe) (5) does not include *M. oryzae* (not present in NW Europe) (6) unknown whether it will comprise *M. graminicola* (not present in NW Europe) | | | | | | | |

**Table 3 - LSU ribosomal DNA-based Meloidogyne species identification. Nucleotide numbering is based on the nematode consensus sequence provided in SEQ ID NO:. Either individual nucleotides (SNPs) or nucleotide combinations (OPs) are unique for the relevant Meloidogyne species**

| **Species** | **Species-specific (combinations of) nucleotides** | | | | | | | | | | | | | | | | | | |
|---|---|---|---|---|---|---|---|---|---|---|---|---|---|---|---|---|---|---|---|
| *M.* naasi | 78A | | 147A | | | 274C | 399T | | 422C | | 464T, 465G, 466A (1) | | | | 630T, 633T, 634T | | | 640C | |
| | | | | | | | | | | | | | | | | | | | |
| *M. fallax* | 647T, 655G | | 890T, 895A, 899G, 900A, 901A | | | | | | | | | | | | | | | | |
| *M. minor* | 214C | | 437T | | 466G | | 539T, 540C | | 577G | | 579G, 580G | | 614T | 420C | | 640G | 693C | | |
| *M. chitwoodi* | 417T | | 557T | | 655A | | 806A | | | | | | | | | | | | |
| | | | | | | | | | | | | | | | | | | | |
| *M. hapla* | 76A | 80A, 81A | | 86C | | 126A | 185G | 371A | | 410A | 518A | 534C | 540A, 541T, 542G | | | | 616C, 623C | | |

| | | | | | | | | | | | | | | | | | | | |
|---|---|---|---|---|---|---|---|---|---|---|---|---|---|---|---|---|---|---|---|
| 1. Cross reactivity with *M. trifoliophila* | | | | | | | | | | | | | | | | | | | |

**Table 4 - LSU rDNA-based primers used to detect Meloidogyne species.**

| Target species | Forward | Reverse |
|---|---|---|
| | | |
| *M. chitwoodi* | 5'-GCATTATTTGGTTTGATTTGGA | 5'-GCTTTTAGGTTTTAAACACCCAAT |
| *M. fallax* | 5'-GCATTATTTGGTTTGATTTGGG | 5'-TGGGGCCATGCTGCTTCCTTTTA |
| *M. minor* | 5'-TCTTGCAAGTATTCATTTACTTTCC | 5'- CAAAACAAAAAATGCTAACACCAC |
| *M. naasi* | 5'-CCAGATTGGGACAGAGTTGA | 5'-ACGATCCACGTAATGAACGA |
| *M. hapla* | 5'-AAGATGGATTTGCAACCAATG | 5'-CAAAAAATGCACGTAAGCCG |

### DNA extraction and real-time amplification

Nematodes were transferred to a 0.2 ml PCR tube containing 25 µl sterile water. An equal volume of lysis buffer containing 0.2 M NaCl, 0.2 M Tris-HCl (pH 8.0), 1% (v/v) β-mercaptoethanol and 800 µg/ml proteinase-K was added. Lysis took place in a Thermomixer (Eppendorf, Hamburg, Germany) at 65 °C and 750 rpm for 2 hrs followed by 5 min. incubation at 100 °C. Lysate was used immediately, or stored at -20°C.

To distinguish between closely-related Meloidogyne species on the basis of single or oligo-nucleotide polymorphisms in LSU-rDNA sequences, real time PCR was performed on a MiniOpticon2 thermal cycler (Bio-Rad, Hercules, CA). Before amplification lysate was diluted 50 times in double-distilled (dd) water. Five µl of diluted template solution was supplemented with 1 ul 5µM forward and 1 µl 5µM reverse primer (Eurogentec, Seraing, Belgium), 10 µl iTaq SybrGreen Supermix (Biorad) and 3 µl water (for primer sequences, see table 4).

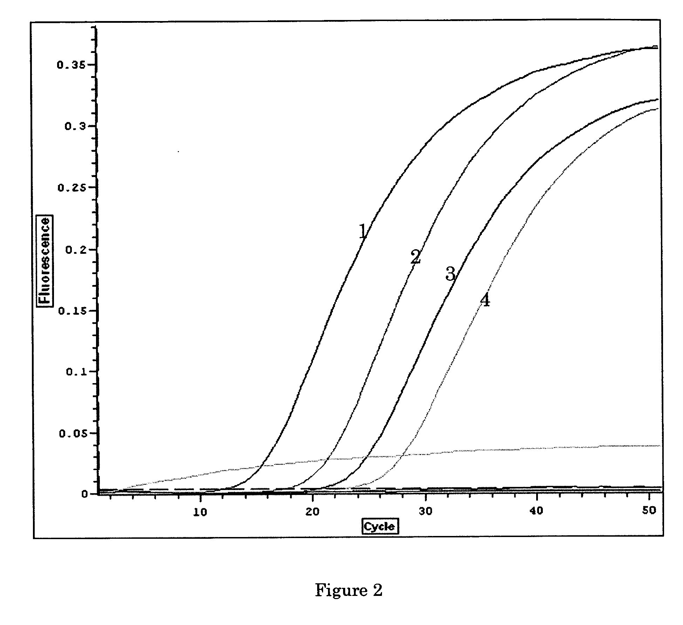

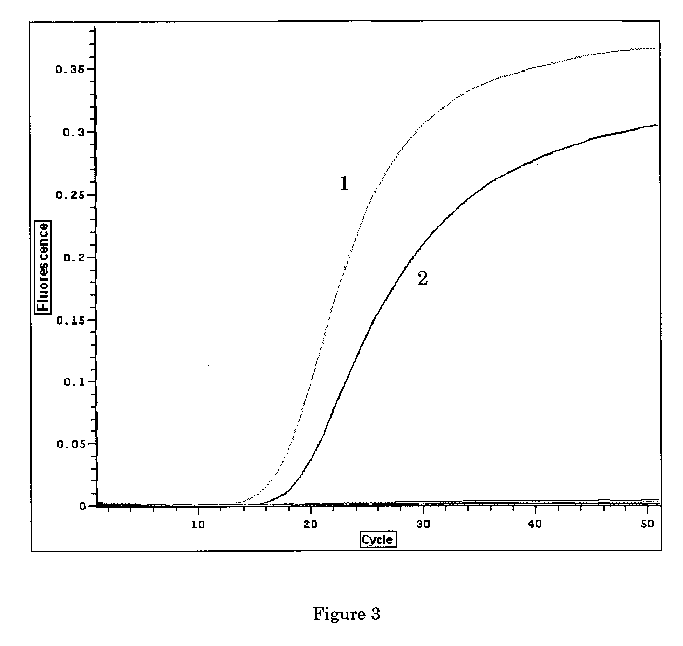

The following PCR profile was used: 95°C for 3 min. followed by 50 x (95°C, 10 sec.; 60°C, 60 sec.; 72°C, 20 sec.). Amplification data were analysed using Biorad MiniOpticon2 software. The results are displayed in Figures 1-3

## Claims

1. A method of testing a sample for the presence of a meloidogyne nematode, said method comprising the step of analyzing the nucleic acid of a sample for the presence of a gene encoding a ribosomal RNA of a meloidogyne nematode or the transcription product thereof having a group-specific single nucleotide polymorphism (SNP) and/or a group-specific oligonucleotide polymorphism (OP), wherein said group comprises at least one species of a meloidogyne nematode of interest.

2. The method of claim 1, wherein said species of interest is selected from *M. arenaria, M. chitwoodi, M. fallax, M. hapla, M. incognita, M. javanica, M. minor* and *M. naasi.*

3. The method of claim 1, wherein said group-specific polymorphism is present in the ribosomal DNA sequence of at least *M. chitwoodi, M. fallax* and *M. minor,* and absent in at least *M. arenaria, M. hapla, M. incognita, M. javanica* and *M. naasi.*

4. The method of claim 1, wherein said group-specific polymorphism is present in the ribosomal DNA sequence of at least *M. naasi, M. oryzae* and *M. graminicola,* and wherein said group-specific polymorphism is absent in the ribosomal DNA sequence of at least *M. arenaria, M. chitwoodi, M. fallax, M. hapla, M. incognita, M. javanica* and *M. minor.*

5. The method of claim 1, wherein said group-specific polymorphism is present in the ribosomal DNA sequence of at least *M. hapla, M. microtyla, M. ardenensis, M. maritime* and *M duytsi,* and wherein said group-specific polymorphism is absent in the ribosomal DNA sequence of at least *M. arenaria, M. chitwoodi, M. fallax, M. incognita, M. javanica, M. minor* and *M. naasi.*

6. The method of claim 1, wherein said group-specific polymorphism is present in the ribosomal DNA sequence of at least *M incognita, M. arenaria* and *M. javanica,* and wherein said group-specific polymorphism is absent in the ribosomal DNA sequence of at least *M. chitwoodi, M. fallax, M. hapla, M. minor* and *M. naasi.*

7. The method of claim 1, wherein the group-specific single nucleotide polymorphisms (SNPs) and/or a group-specific oligonucleotide polymorphisms (OPs) are polymorphisms in the small subunit (SSU) ribosomal RNA gene sequence.

8. The method of claim 7, wherein
(a) said group comprises the species *M. chitwoodi, M. fallax* and *M. minor,* and wherein said polymorphism is selected from the group consisting of:
- OP: 141C, 142A, 143C, 151A, 152A, 153G of SEQ ID NO: 1;
- OP : 192T, 193C, 194T, 199T, 200T, 202T of SEQ ID NO: 2;
- SNP: 677A of SEQ ID NO: 3; and
- OP: 1673G, 1677T of SEQ ID NO: 4;
and/or
(b) said group comprises the species *M. naasi, M. oryzae* and *M. graminicola,* and wherein said polymorphism is selected from the group consisting of:
- OP: 192T, 193T, 194T of SEQ ID NO: 5;
- SNP: 664T of SEQ ID NO: 6;
- OP: 1043A, 1045T, 1046A, 1049A of SEQ ID NO: 7;
- OP: 1353T, 1354A, 1355T, 1356A, 1357A of SEQ ID NO: 8;
- SNP: 1360G of SEQ ID NO: 8; and
- OP: 1702T, 1703T, 1704T of SEQ ID NO: 9;
and/or
(c) said group comprises the species *M. hapla, M. microtyla, M. ardenensis, M. maritime* and *M. duytsi,* and wherein said polymorphism is selected from the group consisting of:
- SNP: 458A of SEQ ID NO: 10; and
- OP: 1331C, 1340C, 1341T, 1342G of SEQ ID NO: 11;
and/or
(d) said group comprises the species *M. incognita, M. arenaria* and *M. javanica,* and wherein said polymorphism is selected from the group consisting of:
- OP: 617C, 620T, 623A, 626A of SEQ ID NO: 12;
- SNP: 1041A of SEQ ID NO: 13;
- OP: 1348A, 1349T, 1350A, 1351C, 1354C, 1355T, 1356T of SEQ ID NO: 14; and
- OP: 1677C, 1679T, 1682A, 1683T, 1684T, 1685T of SEQ ID NO: 15; and wherein the presence of said polymorphism in said gene or said transcription product is indicative of the presence in said sample of at least one member of said group.

9. The method of any one of the preceding claims, wherein said testing comprises:
(a) hybridizing an oligonucleotide comprising a nucleotide sequence complementary to at least a part of the sequence of the rRNA gene of each member of said group, a transcription product thereof, or a complementary nucleic acid thereof under stringent conditions to said nucleic acid, wherein said oligonucleotide is complementary in the position of at least one polymorphism specific for said group, and
(b) detecting whether hybridization has occurred, wherein hybridization of said oligonucleotide is indicative for the presence of at least one member of said group of the meloidogyne nematode.

10. The method of claim 9, wherein said oligonucleotide is detectably labelled.

11. The method of claim 10, wherein said oligonucleotide is labelled with a reporter dye and a quenching dye.

12. The method of any one of the preceding claims, wherein said testing comprises determining the nucleic acid sequence of at least a portion of said a gene encoding the rRNA of a nematode or its transcription product that comprises the group-specific polymorphism.

13. The method of any one of the preceding claims, further comprising amplifying at least a portion of said nucleic acid, preferably prior to analyzing the nucleic acid.

14. The method of any of claims 9-13, wherein said oligonucleotide is an amplification primer and wherein the detection of hybridization in step (b) comprises performing a nucleic acid amplification reaction with said amplification primer.

15. Method according to claim 14, wherein said nucleic acid amplification reaction is quantitative PCR, preferably "real time" quantitative PCR.

16. Method according to claim 15, wherein the reaction is performed by using 'locked' nucleic acids fluorescent probes.

17. The method of any one of the preceding claims, wherein the nucleic acid is analysed by the use of:
(a) a primer extension assay;
(b) a Taqman® PCR;
(c) a differential hybridization assay;
(d) an assay which detects allele-specific enzyme cleavage; and/or
(e) allele-specific PCR.

18. The method of any one of the preceding claims, wherein prior to or instead of analyzing the nucleic acid, at least a portion of said nucleic acid is amplified by a nucleic acid amplification reaction, the reaction comprising cycles of
(a) stringent hybridization with at least two primers sufficiently complementary to bind to said nucleic acid under stringent conditions of hybridization, wherein at least one primer is complementary to the SSU rRNA gene of a group of meloidogyne nematodes comprising at least one species of interest or of its corresponding transcription product or a complement thereof in the position of at least one polymorphism that is specific for said group, and
(b) strand elongation and denaturing,
wherein failure to generate an amplification product is indicative for the absence in said sample of a member of said group of the meloidogyne nematode comprising the polymorphism, and the generation of an amplification product is indicative for the presence in said sample of a member of said group of the meloidogyne nematode comprising the polymorphism.

19. The method of any one of the preceding claims, wherein said sample is a soil sample.

20. The method of any one of the preceding claims, wherein said sample is a root or tuber sample.

21. The method of any one of the preceding claims, wherein said sample is a nucleic acid sample isolated from a nematode.

22. A method of testing a sample for the presence of a meloidogyne nematode species of interest, said method comprising the step of:
- analyzing the nucleic acid of a sample for the presence of a gene encoding a ribosomal RNA of a group of meloidogyne nematode or the transcription product thereof having a group-specific single nucleotide polymorphism (SNP) and/or a group-specific oligonucleotide polymorphism (OP), wherein said group comprises said species of a meloidogyne nematode of interest as a member, and
- analyzing the nucleic acid of said sample for the presence of a gene encoding a ribosomal RNA of a meloidogyne nematode species or the transcription product thereof having a single nucleotide polymorphism (SNP) and/or a oligonucleotide polymorphism (OP) that is specific to said meloidogyne nematode species of interest.

23. Method according to claim 22, wherein said meloidogyne nematode species of interest is selected from the species *M. chitwoodi, M. fallax, M. minor, M. naasi* and *M.hapla.*

24. Method according to claim 23, wherein:
(a) the presence of *M. chitwoodi* is indicated by the presence of a species-specific SNP in the LSU rRNA gene or its transcription product selected from the group consisting of:
- SNP: 417T of SEQ ID NO: 16;
- SNP: 557T of SEQ ID NO: 17;
- SNP: 655A of SEQ ID NO: 18;
- SNP: 806A of SEQ ID NO: 19;
(b) the presence of *M. fallax* is indicated by the presence of a species-specific OP in the LSU rRNA gene or its transcription product selected from the group consisting of:
- OP: 647T, 655G of SEQ ID NO: 20; and
- OP: 890T, 895A, 899G, 900A, 901A of SEQ ID NO: 21;
(c) the presence of *M. minor* is indicated by the presence of a species-specific SNP and/or a species-specific OP in the LSU rRNA gene or its transcription product selected from the group consisting of:
- SNP: 214C of SEQ ID NO: 22;
- SNPs: 420C; 437T and 466G of SEQ ID NO: 23;
- OP: 539T, 540C of SEQ ID NO: 24;
- SNP: 577G of SEQ ID NO: 24;
- OP: 579G, 580G of SEQ ID NO: 24;
- SNPs: 614T and 640G of SEQ ID NO: 25; and
- SNP: 693C of SEQ ID NO: 26;
(d) the presence of *M. naasi* is indicated by the presence of a species-specific SNP and/or a species-specific OP in the LSU rRNA gene or its transcription product selected from the group consisting of:
- SNP: 78A of SEQ ID NO: 27;
- SNP: 147A of SEQ ID NO: 28;
- SNP: 274C of SEQ ID NO: 29;
- SNPs: 399T and 422C of SEQ ID NO: 30;
- OP: 464T, 465G, 466A of SEQ ID NO: 31;
- OP: 630T, 633T, 634T of SEQ ID NO: 32; and
- SNP: 640C of SEQ ID NO: 32;
and/or
(e) the presence of *M. hapla* is indicated by the presence of a species-specific SNP and/or a species-specific OP in the LSU rRNA gene or its transcription product selected from the group consisting of:
- SNPs: 76A; 86C and 126A of SEQ ID NO: 33;
- OP: 80A, 81A of SEQ ID NO: 33;
- SNP: 185G of SEQ ID NO: 34;
- SNPs: 371A and 410A of SEQ ID NO: 35;
- SNPs: 518A; and 534C of SEQ ID NO: 36;
- OP: 540A, 541T, 542G of SEQ ID NO: 36; and
- OP: 616C, 623C of SEQ ID NO: 37.

25. The method of any of claim 22-24, wherein said analysis for the group-specific rRNA gene or its transcription product is performed according to any one of claims 1-21, and wherein said analysis for the species-specific rRNA gene or its transcription product comprises:
(a) hybridizing an oligonucleotide comprising a nucleotide sequence complementary to at least a part of the sequence of the rRNA gene of the meloidogyne nematode species of interest, a transcription product thereof, or a complementary nucleic acid thereof under stringent conditions to said nucleic acid, wherein said oligonucleotide is complementary in the position of at least one polymorphism specific for said meloidogyne nematode species of interest, and
(b) detecting whether hybridization has occurred, wherein hybridization of said oligonucleotide is indicative for the presence of said species of the meloidogyne nematode of interest.

26. The method of claim 25, wherein said oligonucleotide is detectably labelled.

27. The method of claim 26, wherein said oligonucleotide is labelled with a reporter dye and a quenching dye.

28. The method of any of claims 25-27, wherein said oligonucleotide is an amplification primer and wherein the detection of hybridization in step (b) comprises performing a nucleic acid amplification reaction with said amplification primer.

29. Method according to claim 28, wherein said nucleic acid amplification reaction is quantitative PCR, preferably "real time" quantitative PCR.

30. Method according to claim 29, wherein the reaction is performed by using 'locked' nucleic acids fluorescent probes.

31. The method of any one of claims 22-30, wherein the hybridization signal obtained for the positive detection of a member of said group of the meloidogyne nematode when performed by a method of any one of claims 9-21 is compared to the hybridization signal obtained for a positive detection of a particular species of the meloidogyne nematode that is a member of said group when performed by a method of any one of claims 25-30, and wherein the presence of substantially equal hybridization signals confirms of the presence of the species in the sample.

32. An oligonucleotide as defined in any one of the claims 8-30, wherein said oligonucleotide is complementary in the position of at least one group-specific polymorphism as defined in claim 7 and/or at least one species-specific polymorphism as defined in claim 23.

33. A kit of parts suitable for performing a method according to any one of the claims 1-31 comprising an oligonucleotide according to claim 32 and optionally one or more parts selected from the group consisting of nucleic acid extraction means, nucleic acid amplification means, nucleic acid hybridization means, nucleic acid ligation means and nucleic acid detection means.
